# FASCICULE DE BREVET EUROPEEN

(11) **EP 1 745 785 B1**
(45) Date de publication et mention de la délivrance du brevet: **09.03.2011**
(21) Numéro de dépôt: 06291166.4
(22) Date de dépôt: 18.07.2006
(51) Int. Cl.: A61K 31/536, A61K 31/517, A61P 43/00

(54) **Composition pharmaceutique destinée á la prévention ou au traitement des oedèmes cérèbraux**
Pharmazeutische Zusammensetzungen zur Verhütung oder Behandlung von Hirnödemen
Pharmaceutical composition for preventing or treating cerebral oedema

(30) Priorité: 19.07.2005 FR 0507649
(43) Date de publication de la demande: 24.01.2007
(73) Titulaire: Biocodex, 94250 Gentilly (FR)
(72) Inventeur: Le Guern, Marie-Emmanuelle, 60200 Compiegne (FR); Girard, Philippe, 60280 Margny-Les-Compiegne (FR); Gillardin, Jean-Marie, 60680 Jonquieres (FR); Berthon-Cedille, Laurence, 60490 Ricquebourg (FR); Hublot, Bernard, 60200 Compiegne (FR)
(74) Mandataire: Vial, Lionel

(56) Documents cités:
- DE-A1- 3 439 055
- US-A1- 2004 209 904
- LANDOLT AM: "Das Hirnödem" THERAPEUTISCHE UMSCHAU 1973, vol. 30, no. 8, 1973, pages 591-596, XP009063608
- DATABASE EMBASE [Online] ELSEVIER SCIENCE PUBLISHERS, AMSTERDAM, NL; 1985, KRUSE H J ET AL: "Etifoxine: Evaluation of its anticonvulsant profile in mice in comparison with sodium valproate, phenytoin and clobazam" XP002372434 Database accession no. EMB-1985045795 & ARZNEIMITTEL-FORSCHUNG/DRUG RESEARCH 1985 GERMANY, vol. 35, no. 1, 1985, pages 133-135,
- DATABASE EMBASE [Online] ELSEVIER SCIENCE PUBLISHERS, AMSTERDAM, NL; 1986, KRUSE H J ET AL: "Potentiation of clobazam's anticonvulsant activity by etifoxine, a non-benzodiazepine tranquilizer, in mice. Comparison studies with sodium valproate" XP002372435 Database accession no. EMB-1986230303 & ARZNEIMITTEL-FORSCHUNG/DRUG RESEARCH 1986 GERMANY, vol. 36, no. 9, 1986, pages 1320-1322,

## Description

La présente invention concerne une composition pharmaceutique destinée à la prévention ou au traitement de l'oedème cérébral.

L'oedème cérébral est caractérisé comme étant une accumulation excessive d'eau dans les compartiments intra et/ou extracellulaires du cerveau (Pollay (1996) In Neurosurgery, 2ème éd. Mc Graw Hill Book Co., New York, 335-344). L'oedème cérébral peut être d'origine neurologique, comme dans les cas d'attaques ischémiques, d'hémorragies intracérébrales, de tumeurs du cerveau, de méningites ou d'encéphalites, ou bien d'origine non neurologique, comme en cas d'acidocétose diabétique, d'acidose lactique, d'encéphalopathie hypertensive, d'hypertension maligne, d'hyponatrémie ou d'un effet de la haute altitude.

La principale conséquence de l'oedème cérébral est une augmentation de la pression liquidienne intracrânienne, aboutissant à une réduction de l'alimentation sanguine du cerveau et la destruction partielle ou totale des tissus cérébraux insuffisamment vascularisés.

Peu de composés sont disponibles pour le traitement pharmacologique des oedèmes cérébraux, parmi les plus usités, on peut citer les composés suivants :
- le mannitol, et dans une moindre mesure le glycérol, sont utilisés comme agents d'osmothérapie ; toutefois, l'administration prolongée de mannitol conduit à un déséquilibre électrolytique qui peut contrebalancer ses effets bénéfiques en provoquant, par exemple des troubles cardiopulmonaires (Davis et al. (1994) J. Neurosci. Nurs. 26:170-174) ;
- les diurétiques, tel que le furosémide, ne sont utilisés qu'en relais pour prolonger l'effet des agents osmotiques ;
- les corticoïdes, notamment les glucocorticoïdes, telle que la dexaméthasone, agissent principalement sur les vaisseaux sanguins et sont donc particulièrement indiqués dans le cas d'oedèmes cérébraux d'origine vasculaire ; *a contrario,* ils ne sont pas recommandés dans le traitement d'oedèmes consécutifs à des ischémies ou des hémorragies ; par ailleurs, des complications systémiques liées aux stéroïdes peuvent conduire à une détérioration de l'état du patient (Rosenberg (2000) In Neurology in clinical practice, 3ème éd. Butterworth Heinmann, Boston, 2:1545-1559) ;
- les barbituriques, moins utilisés que par le passé, semblent agir en provoquant une diminution du niveau d'activité métabolique ; toutefois, ces composés provoquent également une hypotension systémique et des insuffisances pulmonaires.

Par ailleurs, le document Landolt (1973), Therapeutische Umschau 30:591-596 indique que des crises répétées d'épilepsie peuvent provoquer des oedèmes cérébraux et que l'oedème en développement peut avoir pour conséquence des crises d'épilepsie.

L'étifoxine, ou chlorhydrate de 6-chloro-2-éthylamino-4-méthyl-4-phényl-4H-[3,1]benzoxazine appartient à la famille des benzoxazines. Elle favorise la transmission gabaergique en se liant à un récepteur du canal chlore et est actuellement utilisée comme anxiolytique. Peu de manifestations indésirables consécutives à son utilisation sont répertoriées.

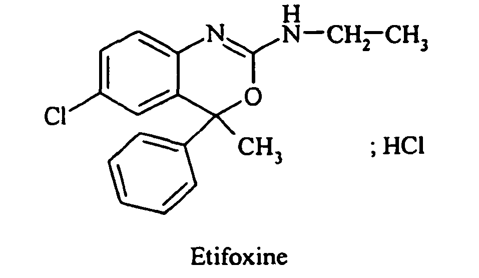

La synthèse de ce composé est notamment décrite dans le brevet français n° 1 571 287. Par ailleurs, plusieurs métabolites actifs de l'étifoxine ont été décrits, tels que la déséthyl-étifoxine ou 2-amino-6-chloro-4-méthyl-4-phényl-4H-[3,1]benzoxazine, la 6-chloro-4-(4-hydroxyphényl)-4-méthyl-3,4-dihydro-1H-quinazolin-2-one ou la 6-chloro-3-éthyl-7-hydroxy-4-méthyl-4-phényl-3,4-dihydro-1H-quinazolin-2-one.

L'objet de la présente invention est de fournir de nouveaux composés, dépourvus des inconvénients des composés déjà connus, dans le cadre de la prévention ou du traitement de l'oedème cérébral.

A ce titre, la présente invention découle de la mise en évidence par les Inventeurs que l'étifoxine et ses dérivés permettent de prévenir ou de traiter l'oedème cérébral.

La présente invention concerne ainsi l'utilisation d'au moins un composé de formule (I) suivante : 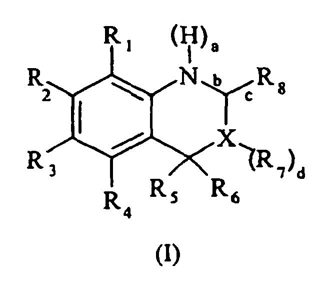 dans laquelle :
- a représente 0 ou 1 ;
- b représente une simple liaison ou une double liaison ;
- c représente une simple liaison ou une double liaison ;
- d représente 0 ou 1 ;
- X représente un atome d'oxygène ou d'azote, sous réserve que lorsque X représente un atome d'oxygène alors d vaut 0 et que lorsque X représente un atome d'azote alors d vaut 1 ;
- R₁, R₂, R₃, et R₄, identiques ou différents, représentent un atome d'hydrogène, un atome d'halogène, notamment choisi parmi F, Cl, Br, ou I, un groupe hydroxyle, ou un groupe alkoxyle de 1 ou 2 atomes de carbone ;
- R₅ et R₆, identiques ou différents, représentent un atome d'hydrogène, un groupe alkyle ou cycloalkyle de 1 à 6 atomes de carbone, ou un groupe aryle de 6 atomes de carbone dont le noyau aromatique est éventuellement substitué par un ou plusieurs atomes d'halogène ou un ou plusieurs groupes hydroxyle, alkoxyle de 1 ou 2 atomes de carbone, trifluorométhyle ou nitro ;
- R₇ représente un atome d'hydrogène, un groupe hydroxyle, ou un groupe alkyle ou hydroxyalkyle de 1 à 3 atomes de carbone ;
- R₈ représente un atome d'oxygène ou un groupe -NR₉R₁₀, R₉ et R₁₀, identiques ou différents, représentant un atome d'hydrogène, un groupe hydroxyle, ou un groupe alkyle ou hydroxyalkyle de 1 à 3 atomes de carbone, sous réserve que lorsque R₈ représente un atome d'oxygène alors a vaut 1, b représente une simple liaison et c représente une double liaison et que lorsque R₈ représente un groupe -NR₉R₁₀ alors a vaut 0, b représente une double liaison et c représente une simple liaison ;
ou de ses sels pharmaceutiquement acceptables,
pour la préparation d'un médicament destiné à la prévention ou au traitement des oedèmes cérébraux.

On désigne par « oedèmes cérébraux » les état dans lesquels il y a une accumulation excessive d'eau dans les compartiments intra et/ou extracellulaires du cerveau. Les oedèmes cérébraux sont notamment décrits dans Pollay (1996) In Neurosurgery, 2ème éd. Mc Graw Hill Book Co., New York, 335-344.

La synthèse des composés de formule (I) définis ci-dessus peuvent aisément être mise en oeuvre à partir des enseignements du brevet français n° 1 571 287.

Il est possible de vérifier l'activité de prévention ou de traitement de l'oedème cérébral des composés de formule (I) ci-dessus, par exemple dans un modèle animal, tel que le rat, en mesurant, après sacrifice, la diminution significative du pourcentage en eau du cerveau d'animaux ayant reçu du triéthylétain et traité par les composés de formule (I), par rapport à des animaux ayant reçu du triéthylétain et non traités. Ce test, décrit par Linee et al., 1984 Ann. Pharm. Fr. 42, 431-442, est mis en oeuvre dans les exemples.

Avantageusement, les composés de formule (I) ci-dessus sont susceptibles d'agir selon un processus différent de celui mis en oeuvre par les composés habituellement utilisés dans le cadre du traitement de l'oedème cérébral et sont donc dépourvus de leurs effets néfastes.

Les sels pharmaceutiquement acceptables selon l'invention apparaîtront de manière évidente pour l'homme du métier, en particulier les sels de chlorhydrate des composés de formule (I) selon l'invention sont préférés.

Comme on l'entend ici, la présente invention concerne également l'utilisation, telle que définie ci-dessus, des formes optiquement actives du composé de formule (I), tels que les énantiomères suivants (lorsque R₅ et R₆ sont différents) : 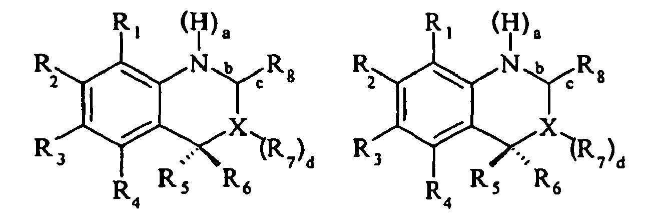 ou leurs mélanges, en particulier leur mélange racémique.

Dans un mode de réalisation particulier, l'invention concerne l'utilisation telle que définie ci-dessus d'un composé de formule (VIII) suivante : 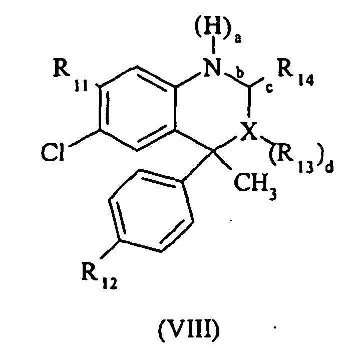 dans laquelle :
- a représente 0 ou 1 ;
- b représente une simple liaison ou une double liaison ;
- c représente une simple liaison ou une double liaison ;
- d représente 0 ou 1 ;
- X représente un atome d'oxygène ou d'azote, sous réserve que lorsque X représente un atome d'oxygène alors d vaut 0 et que lorsque X représente un atome d'azote alors d vaut 1 ;
- R₁₁ et R₁₂ identiques ou différents représentent -H ou -OH ;
- R₁₃ représente -H ou un groupe -CH₂-CH₃ ;
- R₁₄ représente un atome d'oxygène ou un groupe -NH₂ ou -NH-CH₂-CH₃, sous réserve que lorsque R₁₄ représente un atome d'oxygène alors a vaut 1, b représente une simple liaison et c représente une double liaison et que lorsque R₁₄ représente un groupe -NH₂ ou -NH-CH₂-CH₃ alors a vaut 0, b représente une double liaison et c représente une simple liaison.

La présente invention concerne également l'utilisation, telle que définie ci-dessus, des formes optiquement actives du composé de formule (VIII), tels que les énantiomères suivants : 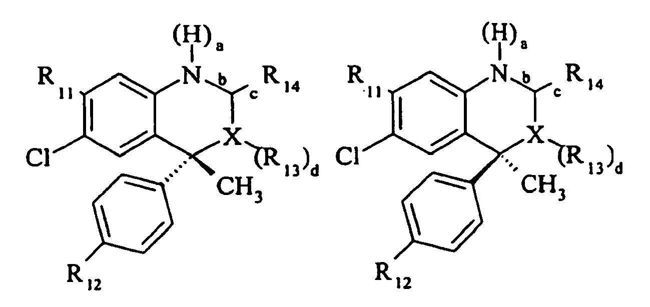 ou leurs mélanges, en particulier leur mélange racémique.

Dans un mode de réalisation particulier, l'invention concerne l'utilisation telle que définie ci-dessus, d'un composé de formule (II) suivante : 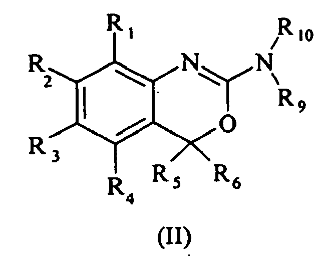 dans laquelle R₁, R₂, R₃, R₄, R₅, R₆, R₉ et R₁₀ sont tels que définis ci-dessus.

La présente invention concerne également l'utilisation, telle que définie ci-dessus, des formes optiquement actives du composé de formule (II), tels que les énantiomères suivants (lorsque R₅ et R₆ sont différents) : 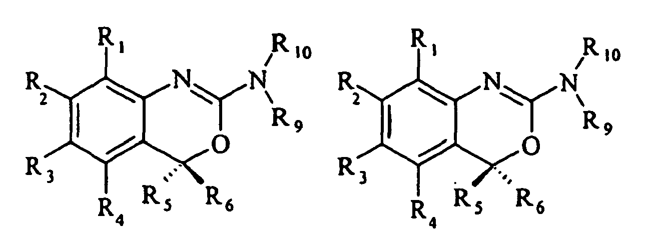 ou leurs mélanges, en particulier leur mélange racémique.

Dans un autre mode de réalisation particulier, l'invention concerne l'utilisation telle que définie ci-dessus, des composés de formules (III) et (IV) suivantes :

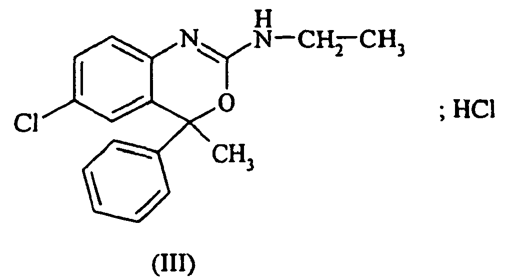

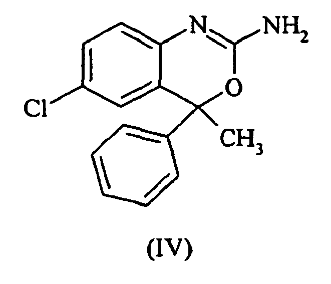

Le composé de formule (III) est l'étifoxine, ou chlorhydrate de 6-chloro-2-éthylamino-4-méthyl-4-phényl-4H-[3,1]benzoxazine.

Le composé de formule (IV), la déséthyl-étifoxine ou 2-amino-6-chloro-4-méthyl-4-phényl-4H-[3,1]benzoxazine, est un métabolite de l'étifoxine.

La présente invention concerne également l'utilisation, telle que définie ci-dessus, des formes optiquement actives du composé de formule (III), tels que les énantiomères suivants : 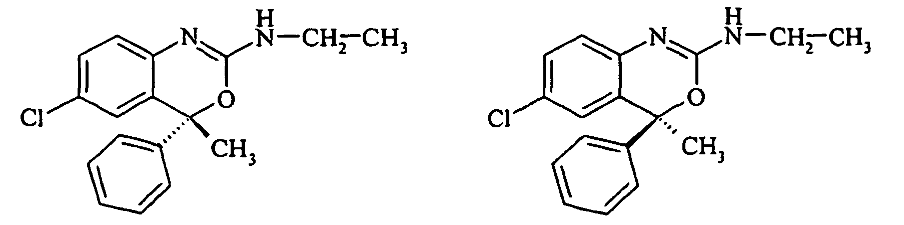 ou leurs mélanges, en particulier leur mélange racémique, notamment sous forme chlorhydrate, ainsi que l'utilisation, telle que définie ci-dessus, des formes optiquement actives du composé de formule (IV), tels que les énantiomères suivants : 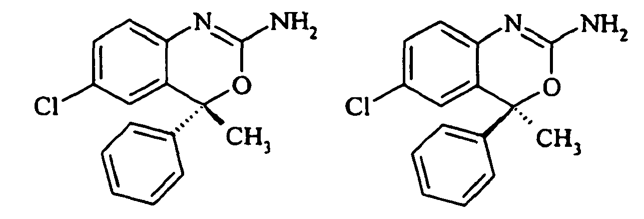 ou leurs mélanges, en particulier leur mélange racémique.

Dans un autre mode de réalisation, l'invention concerne l'utilisation telle que définie ci-dessus, d'un composé de formule (V) suivante : 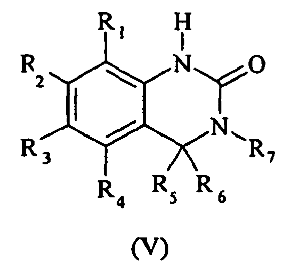 dans laquelle R₁, R₂, R₃, R₅, R₆, et R₇ sont tels que définis ci-dessus.

La présente invention concerne également l'utilisation, telle que définie ci-dessus, des formes optiquement actives du composé de formule (V), tels que les énantiomères suivants (lorsque R₅ et R₆ sont différents) : 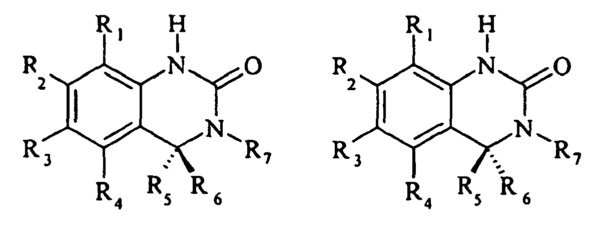 ou leurs mélanges, en particulier leur mélange racémique.

Dans un autre mode de réalisation particulier, l'invention concerne l'utilisation telle que définie ci-dessus, de composés de formules (VI) et (VII) suivantes :

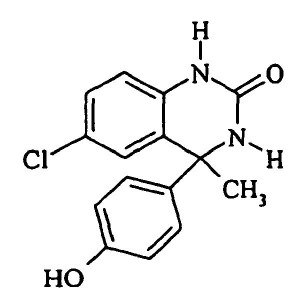

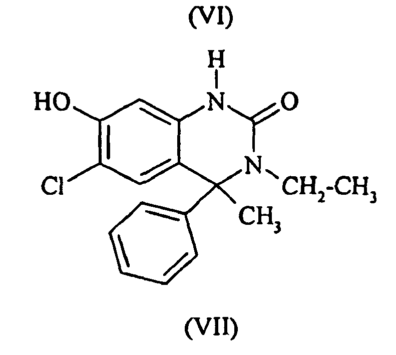

Les composés de formule (VI) (6-Chloro-4-(4-hydroxy-phényl)-4-méthyl-3,4-dihydro-1H-quinazolin-2-one) et (VII) (6-chloro-3-éthyl-7-hydroxy-4-méthyl-4-phényl-3,4-dihydro-1H-quinazolin-2-one) sont des métabolites de l'étifoxine.

La présente invention concerne également l'utilisation, telle que définie ci-dessus, des formes optiquement actives du composé de formule (VI), tels que les énantiomères suivants : 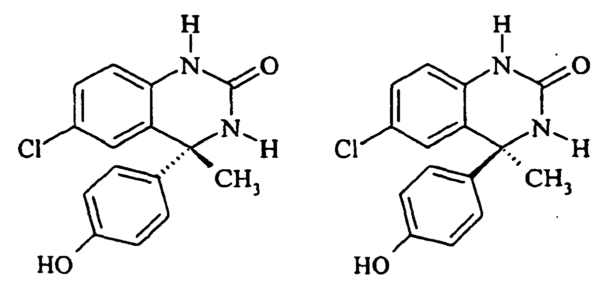 ou leurs mélanges, en particulier leur mélange racémique, ainsi que l'utilisation, telle que définie ci-dessus, des formes optiquement actives du composé de formule (VII), tels que les énantiomères suivants : 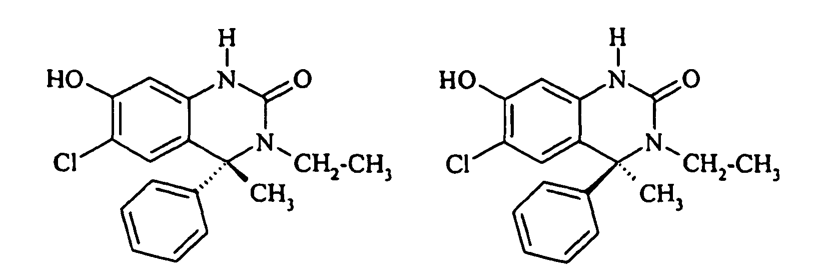 ou leurs mélanges, en particulier leur mélange racémique.

Selon un mode de réalisation particulier de l'utilisation telle que définie ci-dessus, l'oedème cérébral est consécutif à un accident vasculaire cérébral, un traumatisme cérébral, une tumeur cérébrale, des métastases cérébrales d'un cancer, un abcès cérébral, une poussée hypertensive, une acidocétose diabétique ou un neuropaludisme.

Dans un mode de réalisation particulier de l'invention, le médicament défini ci-dessus convient pour l'administration à un individu en ayant besoin d'une dose d'environ 50 mg à environ 1500 mg, notamment d'environ 150 à environ 200 mg, du composé tel que défini ci-dessus.

Dans un mode de réalisation particulier de l'invention, le médicament défini ci-dessus convient pour l'administration à un individu en ayant besoin d'une dose d'environ 50 mg/j à environ 1500 mg/j, notamment d'environ 150 mg/j à environ 200 mg/j, du composé tel que défini ci-dessus.

Selon un autre mode de réalisation préféré, le médicament défini ci-dessus convient pour une administration par voie orale.

Selon un mode de réalisation préféré de l'invention, le médicament défini ci-dessus se présente sous la forme d'une poudre, de cachets, de gélules ou de sachets.

Dans un autre mode de réalisation particulier de l'utilisation telle que définie ci-dessus, le composé défini ci-dessus, est associé à au moins un composé additionnel destiné à la prévention ou au traitement de l'oedème cérébral, tel qu'un composé choisi parmi la liste comprenant un corticoïde, notamment un glucocorticoïde, du glycérol, du mannitol, un diurétique, notamment le furosémide, un barbiturique, le tétracosactide, un antibiotique, la CDP-choline (cytidine 5'-diphosphocholine), la vinpocétine, un inhibiteur calcique, et un antagoniste NMDA (acide N-méthyl-D-aspartique).

Avantageusement, l'association d'un composé de formule (I) ou ses sels pharmaceutiquement acceptables, avec un composé additionnel indiqué dans le traitement de l'oedème cérébral permet de diminuer la dose ou la durée d'administration dudit composé additionnel et ainsi de limiter ses effets secondaires.

La présente invention concerne également une composition pharmaceutique, comprenant à titre de substance active, au moins un composé de formule (I) tel que défini ci-dessus, ou un sel pharmaceutiquement acceptable de celui-ci, et au moins un composé additionnel destiné à la prévention ou au traitement de l'oedème cérébral, tel qu'un composé choisi parmi la liste comprenant un corticoïde, notamment un glucocorticoïde, du glycérol, du mannitol, un diurétique, notamment le furosémide, un barbiturique, le tétracosactide, un antibiotique, la CDP-choline, la vinpocétine, un inhibiteur calcique, un antagoniste NMDA, en association avec un véhicule pharmaceutiquement acceptable.

La présente invention concerne également des produits contenant :
- au moins un composé de formule (I) tel que défini ci-dessus, ou un sel pharmaceutiquement acceptable de celui-ci, et
- au moins un composé additionnel destiné à la prévention ou au traitement de l'oedème cérébral, tel qu'un composé choisi parmi la liste comprenant un corticoïde, notamment un glucocorticoïde, du glycérol, du mannitol, un diurétique, notamment le furosémide, un barbiturique, le tétracosactide, un antibiotique, la CDP-choline, la vinpocétine, un inhibiteur calcique, et un antagoniste NMDA,
comme produit de combinaison pour une utilisation simultanée, séparée ou étalée dans le temps pour la prévention ou le traitement de l'oedème cérébral.

### DESCRIPTION DES FIGURES

### Figure 1A, Figure 1B

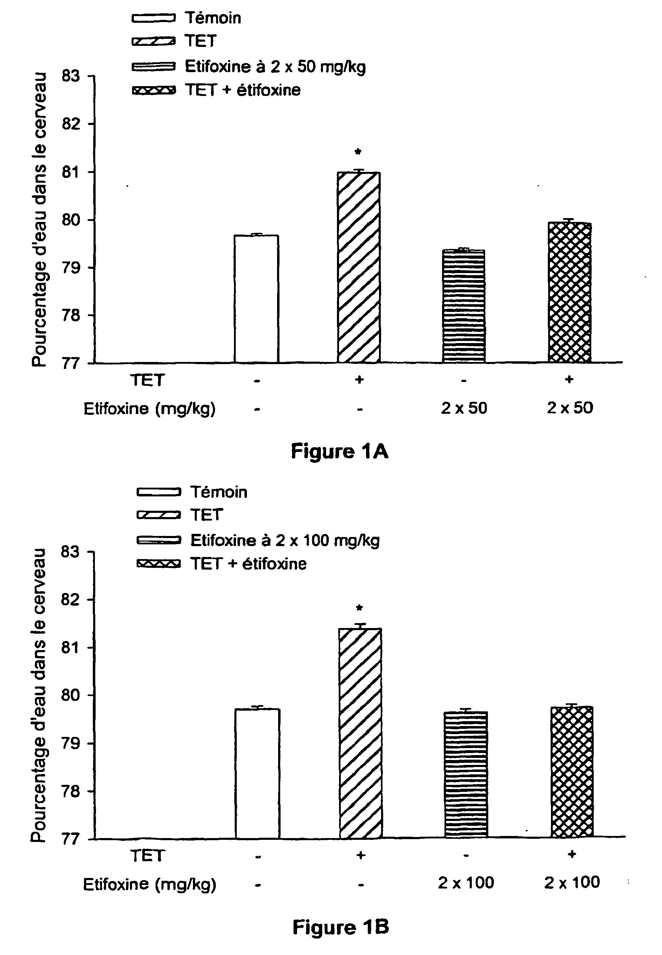

Les Figures 1A et 1B représentent l'effet de l'étifoxine sur l'oedème cérébral induit par le triéthylétain (TET) chez le rat mesuré par le pourcentage d'eau dans le cerveau (axe des ordonnées). Les rats sont répartis par groupes de 10 individus et sont traités sur 5 jours (de J0 à J4).
Figure 1A : les rats ne reçoivent ni TET ni étifoxine (- ; -), du TET en absence d'étifoxine (+ ; -), de l'étifoxine à raison de 50 mg/kg 2 fois par jour en absence de TET (- ; +), ou du TET (3 mg/kg/j) en présence d'étifoxine à raison de 50 mg/kg 2 fois par jour (+ ; +).
Figure 1B : les rats ne reçoivent ni TET ni étifoxine (- ; -), du TET en absence d'étifoxine (+ ; -), de l'étifoxine à raison de 100 mg/kg 2 fois par jour en absence de TET (- ; +) ou du TET (3 mg/kg/j) en présence d'étifoxine à raison de 100 mg/kg 2 fois par jour (+ ; +).
Le symbole étoile ( * ) représente une différence significative par rapport aux groupes sans traitement (p<0,05, ANOVA).

### Figure 2A, Figure 2B

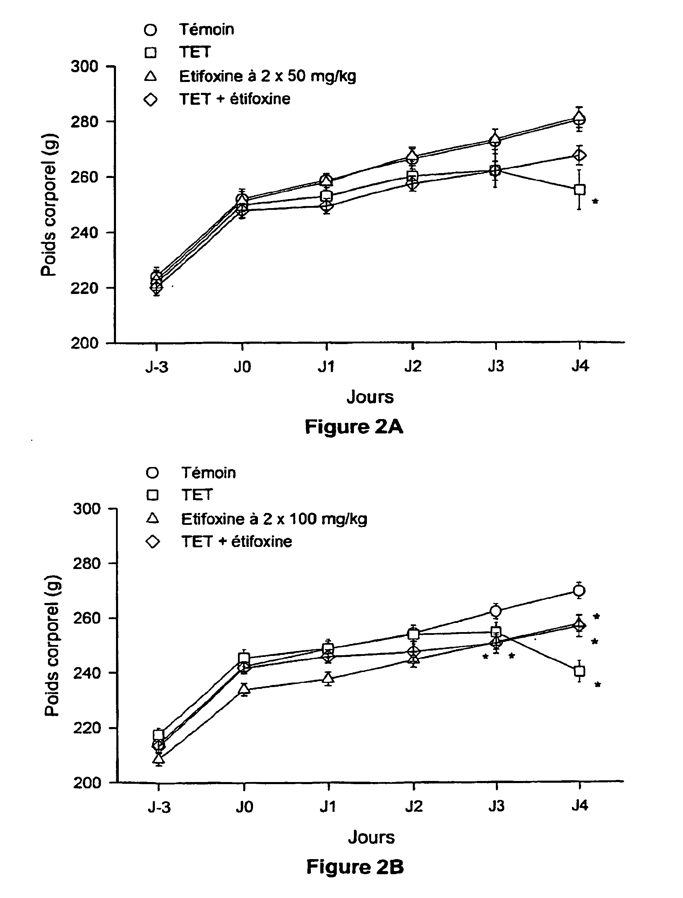

Les Figures 2A et 2B représentent l'effet de l'étifoxine sur l'évolution du poids corporel perturbé par le triéthylétain chez le rat (axe des ordonnées, en grammes) en fonction du temps (axe des abscisses, en jours). Les rats sont répartis par groupes de 10 individus et sont traités sur 5 jours (de J0 à J4).
Figure 2A : les rats ne reçoivent ni TET ni étifoxine (cercles), du TET en absence d'étifoxine (carrés), de l'étifoxine à raison de 50 mg/kg 2 fois par jour en absence de TET (triangles), ou du TET (3 mg/kg/j) en présence d'étifoxine à raison de 50 mg/kg 2 fois par jour (losanges).
Figure 2B : les rats ne reçoivent ni TET ni étifoxine (cercles), du TET en absence d'étifoxine (carrés), de 1' étifoxine à raison de 100 mg/kg 2 fois par jour en absence de TET (triangles) ou du TET (3 mg/kg/j) en présence d'étifoxine à raison de 100 mg/kg 2 fois par jour (losanges).
Le symbole étoile ( * ) représente une différence significative par rapport aux groupes témoins respectifs (p<0,05, ANOVA).

### Figure 3A, Figure 3B

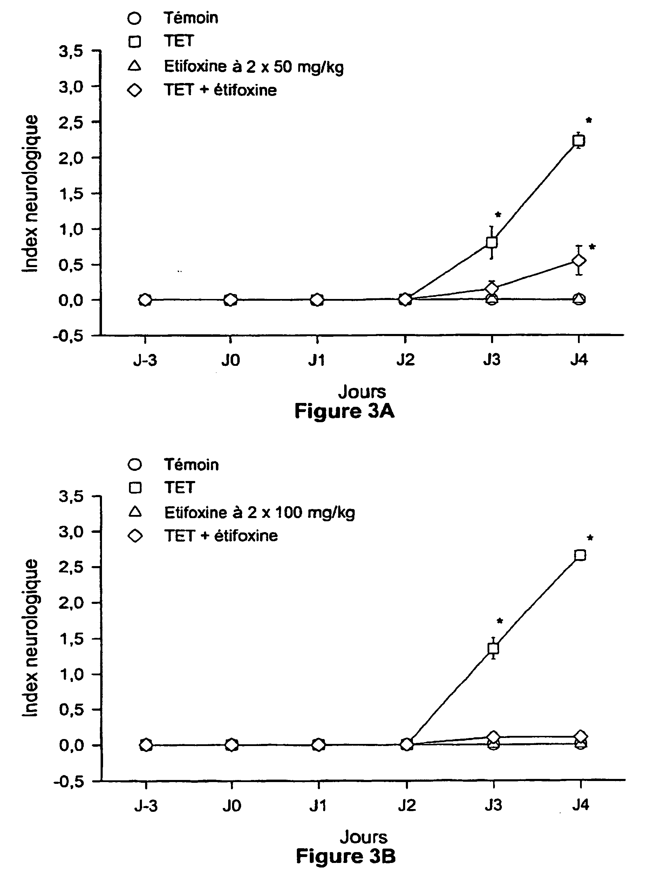

Les Figures 3A et 3B représentent l'effet de l'étifoxine sur l'index neurologique perturbé par le triéthylétain chez le rat (axe des ordonnées) en fonction du temps (axe des abscisses, en jours). Les rats sont répartis par groupes de 10 individus et sont traités sur 5 jours (de J0 à J4).
Figure 3A : les rats ne reçoivent ni TET ni étifoxine (cercles), du TET en absence d' étifoxine (carrés), de l'étifoxine à raison de 50 mg/kg 2 fois par jour en absence de TET (triangles), ou du TET (3 mg/kg/j) en présence d'étifoxine à raison de 50 mg/kg 2 fois par jour (losanges).
Figure 3B : les rats ne reçoivent ni TET ni étifoxine (cercles), du TET en absence d'étifoxine (carrés), de l'étifoxine à raison de 100 mg/kg 2 fois par jour en absence de TET (triangles) ou du TET (3 mg/kg/j) en présence d'étifoxine à raison de 100 mg/kg 2 fois par jour (losanges).
Le symbole étoile ( * ) représente une différence significative par rapport aux groupes témoins respectifs (p<0,05, ANOVA).

### Figure 4A, Figure 4B, Figure 4C

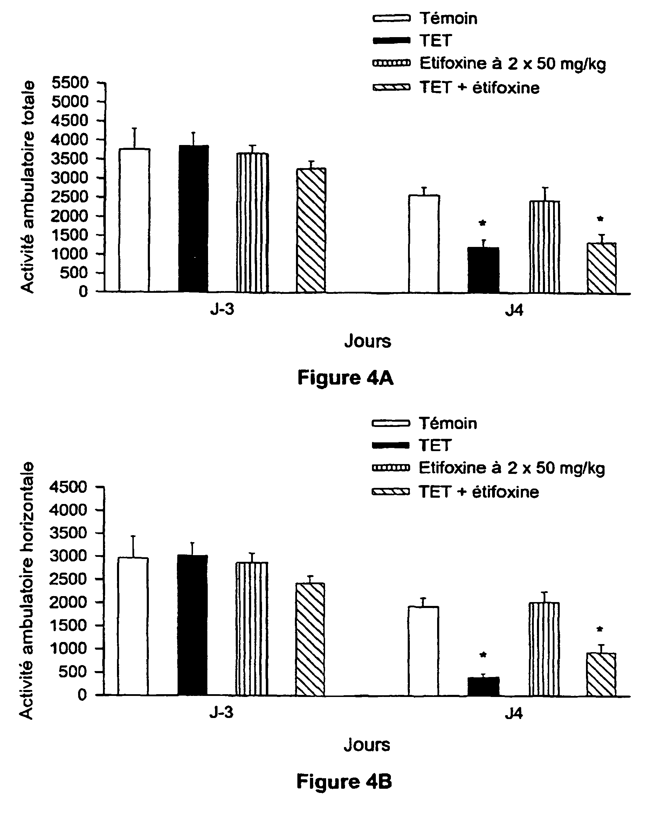

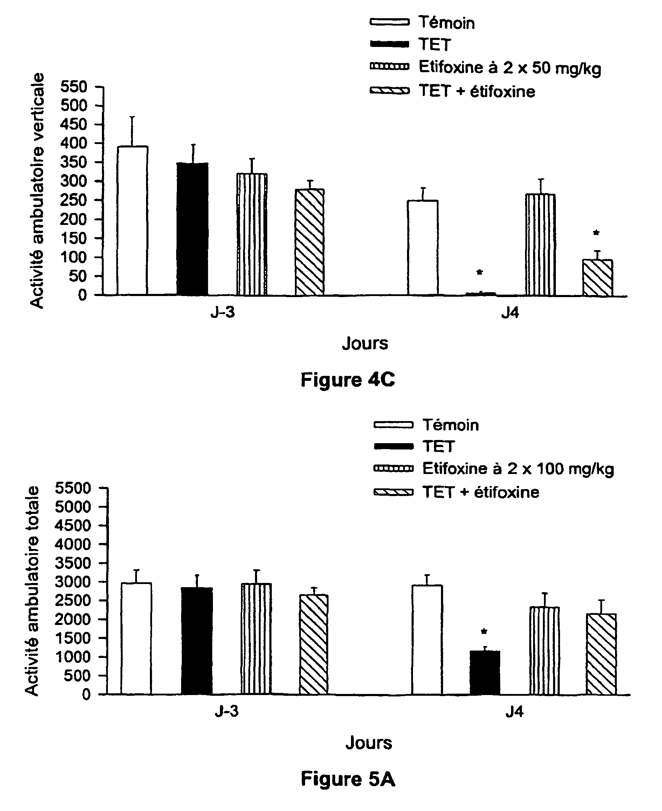

Les Figures 4A, 4B et 4C représentent respectivement l'effet de l'étifoxine sur l'activité ambulatoire (axe des ordonnées, unités arbitraires) totale (Figure 4A), horizontale (Figure 4B) ou verticale (Figure 4C) perturbée par le triéthylétain chez le rat, trois jours avant tout traitement (J-3) ou 5 jours après le début du traitement (J4). Les rats sont répartis par groupes de 10 individus et sont traités sur 5 jours (de J0 à J4).
Les rats ne reçoivent ni TET ni étifoxine (colonne blanche), du TET en absence d'étifoxine (colonne noire), de l'étifoxine à raison de 50 mg/kg 2 fois par jour en absence de TET (colonne hachurée verticalement), ou du TET (3 mg/kg/j) en présence d'étifoxine à raison de 50 mg/kg 2 fois par jour (colonne hachurée obliquement).
Le symbole étoile ( * ) représente une différence significative par rapport aux groupes témoins respectifs (p<0,05, ANOVA).

### Figure 5A, Figure 5B, Figure 5C

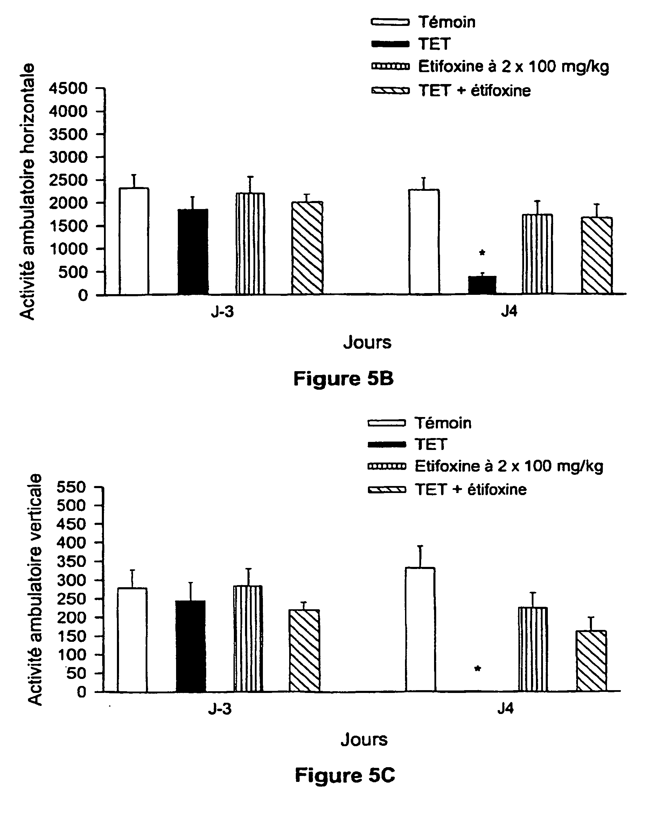

Les Figures 5A, 5B et 5C représentent respectivement l'effet de 1 étifoxine sur l'activité ambulatoire (axe des ordonnées, unités arbitraires) totale (Figure 5A), horizontale (Figure 5B) ou verticale (Figure 5C) perturbée par le triéthylétain chez le rat, trois jours avant tout traitement (J-3) ou 5 jours après le début du traitement (J4). Les rats sont répartis par groupes de 10 individus et sont traités sur 5 jours (de J₀ à J₄).
Les rats ne reçoivent ni TET ni étifoxine (colonne blanche), du TET en absence d'étifoxine (colonne noire), de l'étifoxine à raison de 100 mg/kg 2 fois par jour en absence de TET (colonne hachurée verticalement), ou du TET (3 mg/kg/j) en présence d'étifoxine à raison de 100 mg/kg 2 fois par jour (colonne hachurée obliquement).
Le symbole étoile ( * ) représente une différence significative par rapport aux groupes témoins respectifs (p<0,05, ANOVA).

### Figure 6

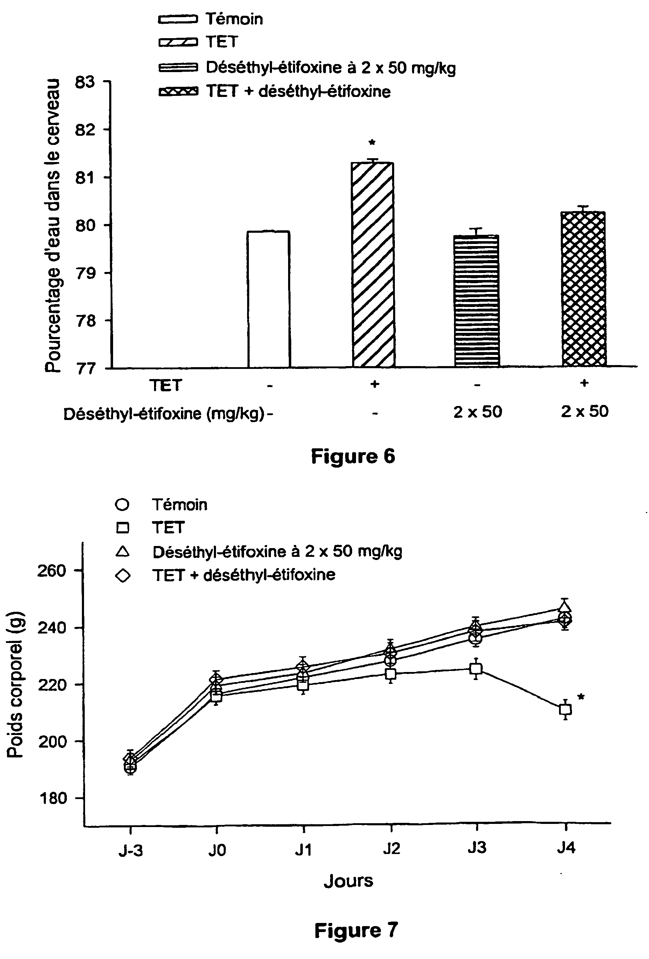

La Figure 6 représente l'effet de la déséthyl-étifoxine sur l'oedème cérébral induit par le triéthylétain (TET) chez le rat mesuré par le pourcentage d'eau dans le cerveau (axe des ordonnées). Les rats sont répartis par groupe de 10 individus et sont traités sur 5 jours (de J0 à J4).
Les rats ne reçoivent ni TET, ni déséthyl-étifoxine (- ; -), du TET (3 mg/kg/j) en absence de déséthyl-étifoxine (+ ; -), de la déséthyl-étifoxine à raison de 50 mg/kg 2 fois par jour en absence de TET (- ; +), ou du TET (3 mg/kg/j) en présence de déséthyl-éifoxine à raison de 50 mg/kg 2 fois par jour (+ ; +).
Le symbole étoile ( * ) représente une différence significative par rapport aux groupes sans traitement (p<0,05, ANOVA).

### Figure 7

La Figure 7 représente l'effet de la déséthyl-etifoxine sur l'évolution du poids corporel perturbé par le triéthylétain chez le rat (axe des ordonnées, en grammes) en fonction du temps (axe des abscisses, en jours). Les rats sont répartis par groupes de 10 individus et sont traités sur 5 jours (de J0 à J4).
Les rats ne reçoivent ni TET ni déséthyl-étifoxine (cercles), du TET (3 mg/kg/j) en absence de déséthyl-étifoxine (carrés) de la déséthyl-étifoxine à raison de 50 mg/kg 2 fois par jour en absence de TET (triangles), ou du TET (3 mg/kg/j) en présence de déséthyl-étifoxine à raison de 50 mg/kg 2 fois par jour (losanges).
Le symbole ( * ) représente une différence significative par rapport aux groupes sans traitement (p < 0,05, ANOVA).

### Figure 8

La Figue 8 représente l'effet de la déséthyl-étifoxine sur l'index neurologique perturbé par le triéthylétain chez le rat (axe des ordonnées) en fonction du temps (axe des abscisses, en jours). Les rats sont répartis par groupes de 10 individus et sont traités sur 5 jours (de J0 à J4). Les rats ne reçoivent ni TET ni déséthyl-étifoxine (cercles), du TET (3 mg/kg/j) en absence de déséthyl-étifoxine (carrés), de la déséthyl-étifoxine à raison de 50 mg/kg 2 fois par jour en absence de TET (triangles), ou du TET (3 mg/kg/j) en présence de déséthyl-étifoxine à raison de 50 mg/kg 2 fois par jour (losanges).
Le symbole étoile ( * ) représente une différence significative par rapport aux groupes sans traitement (p<0,05, ANOVA).

### Figure 9A, Figure 9B, Figure 9C

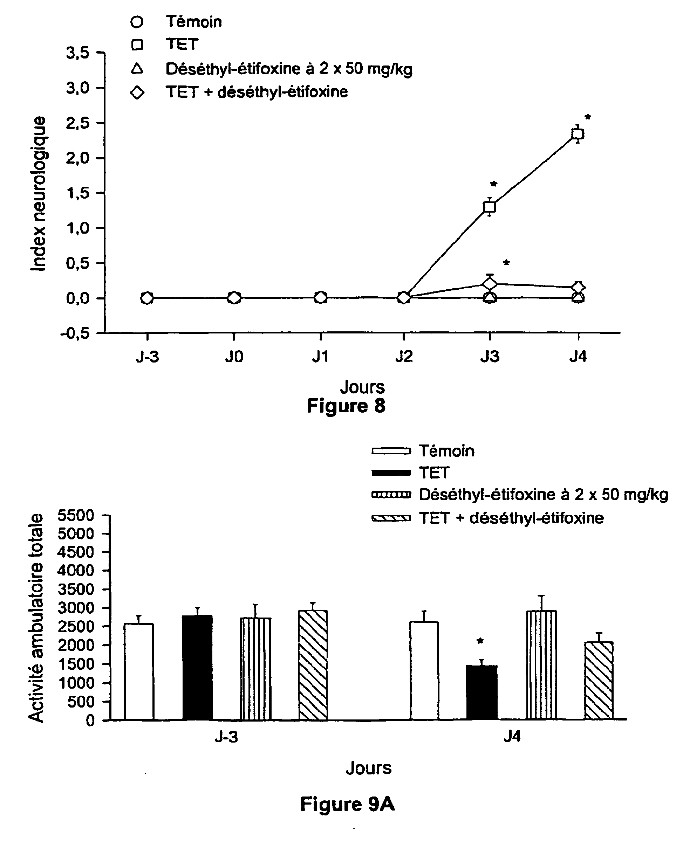

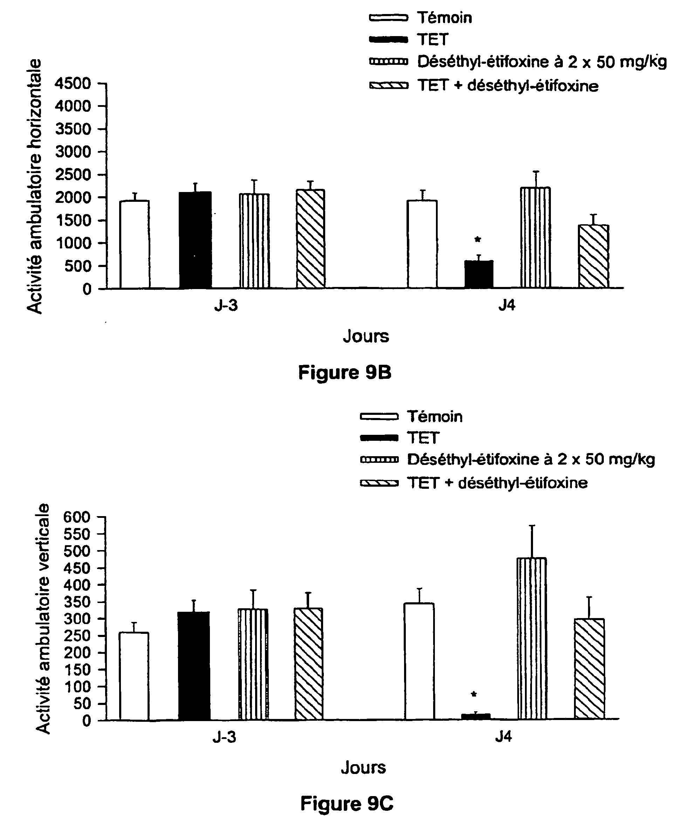

Les Figures 9A, Figure 9B, Figure 9C représentent l'effet de la déséthyl-étifoxine sur l'activité ambulatoire (axe des ordonnées, unités arbitraires) totale (Figure 9A), horizontale (Figure 9B) ou verticale (Figure 9C) perturbée par le triéthylétain chez le rat, trois jours avant tout traitement (J-3) ou 5 jours après le début du traitement (J4). Les rats sont répartis par groupes de 10 individus et sont traités sur 5 jours (de J0 à J4).
Les rats ne reçoivent ni TET ni déséthyl-étifoxine (colonne blanche), du TET (3 mg/kg/j) en absence de déséthyl-étifoxine (colonne noire), de la déséthyl-étifoxine à raison de 50 mg/kg 2 fois par jour en absence de TET (colonne hachurée verticalement), ou du TET (3 mg/kg/j) en présence de déséthyl-étifoxine à raison de 50 mg/kg 2 fois par jour (colonne hachurée obliquement).
Le symbole ( * ) représente une différence significative par rapport aux groupes sans traitement (p<0,05, ANOVA).

### EXEMPLES

Les Inventeurs ont mis en évidence une protection de l' étifoxine sur l'oedème cérébral induit par le chlorure de triéthylétain (TET). Par ailleurs, l'effet de l'étifoxine a également été étudié sur les perturbations induites par le TET pour les 3 paramètres suivants : l'évolution pondérale, l'index neurologique et l'activité ambulatoire.

### 1. MATERIEL ET METHODES

### 1.1. Modèle

L'oedème cérébral induit par le chlorure de triéthylétain (TET) chez le rat est un modèle physiopathologique pour l'étude de substances préconisées dans le traitement de certaines affections cérébrovasculaires (Linee et al., 1984 Ann. Pharm. Fr. 42, 431-442). L'intoxication au TET est également un outil toxicologique utile pour tester des produits agissant au niveau cérébral chez la personne âgée pour tester des nouveaux produits dans la sénescence (Bentue-Ferer et al., 1985 Exp. Aging Res. 11, 137-141).

L'oedème cérébral dû au TET est un oedème chronique, d'apparition progressive et spontanément réversible à condition d'arrêter l'intoxication. Cet oedème se développe exclusivement au niveau du cerveau et de la moelle épinière. L'oedème cérébral se caractérise par une augmentation des teneurs en eau, sodium et chlorures sans modification notable de celle du potassium. L'oedème se traduit par une atteinte spécifique de la substance blanche (Naruse et al., 1982 J. Neurosurg. 56, 747-752), avec un élargissement des espaces intramyéliniques et atteinte de la myéline (Kirschner and Sapirstein, 1982 J. Neurocytol. 11, 559-569). La myéline du système nerveux central possède le potentiel pour récupérer son intégrité après des dommages oedémateux par le retrait du fluide accumulé (Yanagisawa et al., 1990 Neurochem. Res. 15, 483-486). L'importance de l'oedème, qui s'accompagne d'une perte pondérale et de désordres neurologiques périphériques, est proportionnelle à la dose de TET.

Il a été en particulier démontré que :
- L'administration de TET (bromure) à 1 mg/kg/j par voie intrapéritonéale, pendant 7 jours chez le rat, augmente le pourcentage d'eau dans la substance blanche (de 78 % à 82 %), mais pas dans la substance grise (Naruse et al., 1982 J. Neurosurg. 56, 747-752) ;
- L'administration de TET (chlorhydrate) dans l'eau de boisson à 2-3 %, pendant 15 jours chez le rat, augmente le pourcentage d'eau de 78,0 % à 80,0 % (Borzeix and Cahn, 1984 Int. J. Clin. Pharmacol. Res. 4, 259-261) ;
- L'administration de TET (chlorure) à 2 mg/kg/j par voie orale, pendant 5 jours chez le rat, augmente le pourcentage d'eau de 76-77 % à 79-80 % (Linee et al., 1984 Ann. Pharm. Fr. 42, 431-442)
- L'administration de TET (chlorure) à 0.002 % dans l'eau de boisson, pendant 14 jours chez le rat, augmente le pourcentage d'eau de 78,3 % à 81,1 % (Otani et al., 1986 Acta Neuropathol. (Berl) 69, 54-65).

Selon un protocole préventif, les substances à tester sont administrées pendant l'intoxication à l'étain et leurs activités sont mesurées après 5 jours. Dans ces conditions, il a été montré que certains médicaments cérébrovasculaires sont actifs, comme la dihydroergotoxine, la (-)éburnamonine et la vincamine (Linee et al., 1984 Ann. Pharm. Fr. 42, 431-442).

### 1.2. Animaux

Des rats mâles Wistar de chez Janvier de poids compris entre 200 et 250 grammes au début de l'expérience, sont utilisés après au moins 7 jours d'acclimatation dans l'animalerie (t° ambiante = 22 ± 2° C ; hygrométrie relative = 50 ± 20 % ; nourriture UAR "A04" ; cycle nycthéméral (12h/12h : 7h-19h/19h-7h)).

### 1.3. Protocole expérimental

Le protocole a été adapté d'après Linee *et al.* (Linee et al., 1984 Ann. Pharm. Fr. 42, 431-442) :
- à J-3, les rats sont répartis au hasard dans des cages (5 rats par cage) ;
- le triéthylétain est administré par voie orale pendant 5 jours (de J0 à J4) vers 8h-8h30 ;
- le produit étudié est donné par voie orale 2 fois par jour pendant 5 jours (de J0 à J4) vers 9h-9h30 et 16h ;
- on note tous les jours le poids corporel et l'index neurologique ;
- l'activité ambulatoire est mesurée à J-3, avant tout traitement, et à J4, à la fin de l'étude ;
- dès la fin des mesures, le rat est sacrifié par décapitation, et on prélève son cerveau ; chaque cerveau est pesé (poids frais), puis est placé dans une étuve pour obtenir son poids sec.

### 1.4. Expression des résultats

### Teneur en eau du cerveau

- Le cerveau est pesé après le prélèvement pour obtenir le poids frais.
- Le cerveau est ensuite placé dans une étuve pour dessiccation à poids constant à 90° C pendant 72 h, pour obtenir le poids sec.
- On calcule ensuite le pourcentage d'eau de chaque cerveau.

### Index neurologigue : selon les 4 critères suivants

- **0** : pas d'anomalie apparente
- **1** : perte de l'activité spontanée : le rat ne sort pas d'une surface limitée dans un délai de 60 secondes, mais il s'en échappe normalement s'il est stimulé (bruit, pincement) ; il a perdu son activité exploratoire mais conserve ses capacités motrices.
- **2** : perte du réflexe d'agrippement lorsque le rat est repoussé sur la surface.
- **3** : perte du réflexe de retrait, coma suivi dans la plupart des cas de la mort.

### Activité ambulatoire

L'activité motrice est détectée à l'aide de 15 cellules photoélectriques réparties sur les parois de l'enceinte rectangulaire (320x290x100 mm) du système Opto-Varimex de Colombus instruments U.S.A..

Le nombre des déplacements (activités ambulatoire, horizontale et verticale), de l'animal est comptabilisé pendant 15 minutes. L'activité est exprimée en unité arbitraire : 1 unité correspond à un passage devant une cellule photoélectrique.

### 1.5. Produits

Le triéthylétain bromure à 97 % (Sigma, réf 288047) est dilué dans l'eau distillée.

L'étifoxine et la déséthyl-étifoxine sont mises en suspension dans du Tween 80 à 1 % et administrées sous un volume de 0,5 ml pour 100 g de poids corporel.

### 1.6. Statistiques

Le test statistique utilisé est l'analyse de variance (ANOVA). Quand le résultat ne dépend pas du hasard (à 5 %), on détermine les groupes traités qui diffèrent du groupe témoin.

### 2. RESULTATS

### 2.1. Effet dose du triéthylétain

Plusieurs doses de TET ont été étudiées pour déterminer une dose permettant d'obtenir des résultats reproductibles.

L'administration de TET à 2 mg/kg/j par voie orale entraîne assez peu d'effet au niveau de l'évolution du poids corporel des rats, et au niveau de l'index neurologique.

L'administration de TET à 3 mg/kg/j provoque une diminution du poids corporel des rats et des signes de toxicité neurologique dès le 4^{ème} jour, mais pas de mortalité.

L'administration de TET à 4 mg/kg/j entraîne des signes de toxicité neurologique dès le 2^{ème} jour, et de la mortalité dès le 4^{ème} jour.

Par conséquent, la dose de 3 mg/kg/j a été choisie pour les études suivantes.

### 2.2. Effet de l'étifoxine

### Oedème cérébral

L'administration propre d'étifoxine à 2 x 50 ou 2 x 100 mg/kg/j pendant 5 jours ne modifie pas le pourcentage d'eau du cerveau chez le rat (**Tableau 1** et **Figures 1A, 1B**).

Le TET à 3 mg/kg/j, pendant 5 jours, entraîne une augmentation significative du pourcentage d'eau, de 79,65 % et 79,70 % respectivement pour les deux groupes témoins, à 80,96 % et 81,37 % respectivement pour les deux groupes traités, indiquant la présence d'un oedème cérébral.

L'administration concomitante d'étifoxine à 2 x 50 mg/kg/j et de TET supprime l'augmentation significative du pourcentage d'eau induit par le TET. De même, à la dose de 2 x 100 mg/kg/j, l'étifoxine inhibe l'effet du TET sur le pourcentage d'eau.

D'autre part, le poids du cerveau déshydraté est respectivement de 281 et 278 mg dans les groupes témoins. Le TET ou l'étifoxine administrés seuls, ou administrés ensemble, ne modifient pas significativement ce paramètre.

### Poids corporel

Au niveau de l'évolution du poids corporel des rats, le TET entraîne une chute de poids significative à J4 (**Tableau 2** et **Figures 2A, 2B**).

L'étifoxine administrée seule à 2 x 50 mg/kg/j ne perturbe pas la prise de poids. A la dose de 2 x 100 mg/kg/j, l'étifoxine entraîne une diminution significative du poids corporel des rats à J3 et à J4 par rapport aux groupes témoins, mais ce n'est pas une chute comme pour le TET.

En présence de TET et d'étifoxine à 2 x 50 mg/kg/j, on remarque que le poids corporel des rats à J4 n'a pas autant diminué que celui du groupe recevant seulement le TET , et la différence n'est plus significative par rapport au groupe témoin. Dans ce cas, l'étifoxine empêche partiellement la chute de poids induite par le TET.

En présence de TET et d'étifoxine à 2 x 100 mg/kg/j, on peut noter que la chute de poids observée à J4 dans le groupe recevant seulement le TET ne se produit pas. L'étifoxine semble donc diminuer la chute de poids induite par le TET.

### Index neurologique

L'administration d'étifoxine à 2 x 50 ou 2 x 100 mg/kg/j pendant 5 jours n'entraîne aucun signe comportemental chez le rat (**Tableau 3** et **Figures 3A, 3B**).

Le TET à 3 mg/kg/j, pendant 5 jours, entraîne une augmentation significative de l'index neurologique à partir de J3, avec un effet beaucoup plus fort à J4, indiquant des troubles neurologiques importants.

L'administration concomitante d'étifoxine à 2 x 50 mg/kg/j et de TET inhibe l'augmentation de l'index neurologique à J3, et le réduit fortement à J4.

A la dose de 2 x 100 mg/kg/j, l'étifoxine inhibe totalement l'effet du TET sur l'index neurologique.

### Activité ambulatoire

L'administration propre d'étifoxine à 2 x 50 mg/kg/j ou 2 x 100mg/kg/j pendant 5 jours n'entraîne aucun effet significatif sur les activités ambulatoires chez le rat (**Tableaux 4 et 5 et**

### Figures 4A, 4B, 4C, 5A, 5B, 5C).

Le TET à 3 mg/kg/j, pendant 5 jours, entraîne une forte diminution des activités ambulatoires à J4, avec une inhibition presque totale de l'activité ambulatoire verticale.

L'administration concomitante d'étifoxine à 2 x 50 mg/kg/j et de TET ne supprime pas l'effet du TET, mais le réduit partiellement au niveau de l'activité ambulatoire verticale. A la dose de 2 x 100 mg/kg/j, l'étifoxine inhibe totalement l'effet du TET au niveau des activités ambulatoires totale et horizontale et le réduit fortement au niveau de l'activité ambulatoire verticale.

### 2.3. Effet de la déséthyl-étifoxine

### Oedème cérébral

L'administration propre de déséthyl-étifoxine à 2 x 50 mg/kg/j pendant 5 jours ne modifie pas le pourcentage d'eau chez le rat (**Tableau 6** et **Figure 6**).

Le TET à 3 mg/kg/j, pendant 5 jours, entraîne une augmentation significative du pourcentage d'eau, de 79,85 % à 81,29 %, indiquant la présence d'un oedème cérébral.

L'administration concomitante de déséthyl-étifoxine à 2 x 50 mg/kg/j et de TET supprime l'augmentation significative du pourcentage d'eau induit par le TET.

D'autre part, le poids du cerveau déshydraté est de 267 ± 3 mg dans le groupe témoin.

Le TET ou la déséthyl-étifoxine, administrés indépendamment ou administrés ensemble, ne modifient pas significativement ce paramètre.

### Poids corporel

Au niveau de l'évolution du poids corporel des rats, le TET entraîne une chute de poids significative à J4 (**Tableau 7** et **Figure 7**).

La déséthyl-étifoxine administrée seule à 2 x 50 mg/kg/j ne perturbe pas la prise de poids.

En présence de TET et de déséthyl-étifoxine à 2 x 50 mg/kg/j, on remarque que le poids corporel des rat s à J4 n'a pas diminué par rapport au groupe témoin. La déséthyl-étifoxine bloque la chute de poids corporel induite par le TET.

### Index neurologique

L'administration propre de déséthyl-étifoxine à 2 x 50 mg/kg/j pendant 5 jours n'entraîne aucun signe comportemental chez le rat **(Tableau 8** et **Figure 8****).**

Le TET à 3 mg/kg/j, pendant 5 jours, entraîne une augmentation significative de l'index neurologique à partir de J3, avec un effet plus fort à J4, indiquant des troubles neurologiques importants.

L'administration concomitante de déséthyl-étifoxine à 2 x 50 mg /kg/j et de TET inhibe l'augmentation de l'index neurologique à J3 et à J4.

### Activité ambulatoire

L'administration propre de déséthyl-étifoxine à 2 x 50 mg/kg/j pendant 5 jours n'entraîne aucun effet significatif sur les activités ambulatoires chez le rat (**Tableau 9** et **Figures 9A****,** **9B, 9C**).

Le TET à 3 mg/kg/j, pendant 5 jours, entraîne une forte diminution des activités ambulatoires à J4, avec une diminution presque totale de l'activité ambulatoire verticale.

L'administration concomitante de déséthyl-étifoxine à 2 x 50 mg/kg/j et de TET supprime totalement l'effet du TET.

**TABLEAU 1 : Effet de l'étifoxine sur l'oedème cérébral induit par le triéthylétain (TET) en mesurant le pourcentage d'eau du cerveau, chez le rat (n = 10). Le rat reçoit, par voie orale pendant 5 jours, de l'étifoxine 2 fois par jour et du TET à 3 mg/kg/j. Le 5^{ème} jour, le cerveau est prélevé puis desséché pendant 72 heures à 90°C, pour déterminer le pourcentage d'eau présent dans le cerveau.**

| **TET (mg/kg/j)** | **Etifoxine (mg/kg/j)** | **Pourcentage d'eau (moyenne ± esm)** | **Variation en pourcentage** | **Test statistique ANOVA** |
|---|---|---|---|---|
| 0 | 0 | 79,65 ± 0,05 | | |
| 3 | 0 | 80,96 ± 0,07 | + 1,31 | p < 0,05 |
| 0 | 2 x 50 | 79,35 ± 0,05 | - 0,30 | ns |
| 3 | 2 x 50 | 79,92 ± 0,08 | + 0,27 | ns |
| 0 | 0 | 79,70 ± 0,07 | | |
| 3 | 0 | 81,37 ± 0,10 | + 1,67 | p < 0,05 |
| 0 | 2 x 100 | 79,63 ± 0,07 | - 0,07 | ns |
| 3 | 2 x 100 | 79,72 ± 0,07 | + 0,02 | ns |

**TABLEAU 2 : Effet de l'étifoxine sur l'évolution du poids corporel perturbée par le triéthylétain (TET) chez le rat (n = 10). A partir de J0, le rat reçoit, par voie orale pendant 5 jours, de l'étifoxine 2 fois par jour et du TET à 3 mg/kg/j. (*:p < 0,05 par un test statistique ANOVA pour comparer les groupes traités au groupe sans traitement).**

| **TET (mg/kg/j)** | **Etifoxine (mg/kg/j)** | **Poids corporel en grammes (moyenne ± esm)** | | | | | |
|---|---|---|---|---|---|---|---|
| | | **J-3** | **J0** | **J1** | **J2** | **J3** | **J4** |
| 0 | 0 | 224±4 | 252±4 | 259±4 | 266±4 | 273±5 | 281 ±4 |
| 3 | 0 | 222±4 | 250±5 | 253±5 | 260±5 | 262±6 | 255±7* |
| 0 | 2 x 50 | 223±4 | 251±4 | 258±3 | 267±3 | 274±4 | 282±4 |
| 3 | 2 x 50 | 220±3 | 248±3 | 249±3 | 257±3 | 262±3 | 268±4 |
| 0 | 0 | 214±2 | 242±2 | 249±3 | 254±3 | 262±3 | 270±3 |
| 3 | 0 | 218±2 | 246±3 | 249±3 | 254±3 | 255±4* | 240±4* |
| 0 | 2x100 | 209±2 | 234±2 | 238±2 | 245 ±4 | 251 ±3 * | 258±3 * |
| 3 | 2x100 | 213±2 | 242±2 | 246±2 | 248±4 | 251±4 | 257±4 * |

**TABLEAU 3 : Effet de l'étifoxine sur l'index neurologique perturbé par le triéthylétain (TET) en fonction du temps, chez le rat (n = 10). A partir de J0, le rat reçoit, par voie orale pendant 5 jours, de l'étifoxine 2 fois par jour et du TET à 3 mg/kg/j. (*:p<0,05, test statistique ANOVA par rapport aux groupes témoins respectifs).**

| **TET (mg/kg/j)** | **Etifoxine (mg/kg/j)** | **Index neurologique (moyennes ± esm)** | | | | | |
|---|---|---|---|---|---|---|---|
| | | **J-3** | **J0** | **J1** | **J2** | **J3** | **J4** |
| 0 | 0 | 0 | 0 | 0 | 0 | 0 | 0 |
| 3 | 0 | 0 | 0 | 0 | 0 | 0,80±0,23* | 2,25±0,11* |
| 0 | 2 x 50 | 0 | 0 | 0 | 0 | 0 | 0 |
| 3 | 2 x 50 | 0 | 0 | 0 | 0 | 0,15±0,11* | 0,55±0,20* |
| 0 | 0 | 0 | 0 | 0 | 0 | 0 | 0 |
| 3 | 0 | 0 | 0 | 0 | 0 | 1,35±0,15* | 2,65±0,08 * |
| 0 | 2x100 | 0 | 0 | 0 | 0 | 0 | 0 |
| 3 | 2x100 | 0 | 0 | 0 | 0 | 0,10±0,07* | 0,10±0,07* |

| | | | | | | | |
|---|---|---|---|---|---|---|---|
| Index neurologique : 0 →pas d'anomalie apparente 1 → perte de l'activité spontanée : le rat ne sort pas d'une surface limitée dans un délai de 60 secondes, mais il s'en échappe normalement s'il est stimulé (bruit, pincement) ; il a perdu son activité exploratoire mais conserve ses capacités motrices 2 → perte du réflexe d'agrippement lorsque le rat est repoussé sur la surface 3 → perte du réflexe de retrait, coma suivi dans la plupart des cas de la mort | | | | | | | |

**TABLEAU 4 : Effet de l'étifoxine sur l'activité ambulatoire (unité arbitraire) perturbée par le triéthylétain (TET) chez le rat (n = 10). Le rat reçoit, par voie orale pendant 5 jours, de l'étifoxine 2 fois par jour et du TET à 3 mg/kg/j. Les mesures sont effectuées à J-3, avant tout traitement, et à J4, à la fin de l'étude. ( * : p<0,05, test statistique ANOVA par rapport aux groupes témoins respectifs).**

| **TET** **(mg/kg/j)** | **Etifoxine** **(mg/kg/j)** | **J-3** **(moyenne ± esm)** | **J4** **(moyennes ± esm)** |
|---|---|---|---|
| **ACTIVITE AMBULATOIRE TOTALE** | | | |
| 0 | 0 | 3758 ± 537 | 2569 ± 204 |
| 3 | 0 | 3859 ± 333 | 1208 ± 195 * |
| 0 | 2 x 50 | 3660 ± 212 | 2434 ± 346 |
| 3 | 2 x 50 | 3260 ± 196 | 1339 ± 226 * |

| **ACTIVITE AMBULATOIRE HORIZONTALE** | | | |
|---|---|---|---|
| 0 | 0 | 2964 ± 467 | 1925 ± 187 |
| 3 | 0 | 3028 ± 273 | 405 ± 80 * |
| 0 | 2 x 50 | 2874 ± 202 | 2018 ± 223 |
| 3 | 2 x 50 | 2429 ± 159 | 934 ± 180 * |

| **ACTIVITE AMBULATOIRE VERTICALE** | | | |
|---|---|---|---|
| 0 | 0 | 391 ± 80 | 250 ± 33 |
| 3 | 0 | 348 ± 49 | 7 ± 3 * |
| 0 | 2 x 50 | 322 ± 40 | 268 ± 39 |
| 3 | 2 x 50 | 281 ± 23 | 98 ± 23 * |

**TABLEAU 5 : Effet de l'étifoxine sur l'activité ambulatoire (unité arbitraire) perturbée par le triéthylétain (TET) chez le rat (n = 10). Le rat reçoit, par voie orale pendant 5 jours, de l'étifoxine 2 fois par jour et du TET à 3 mg/kg/j. Les mesures sont effectuées à J-3, avant tout traitement, et à J4, à la fin de l'étude. ( * : p<0,05, test statistique ANOVA par rapport aux groupes témoins respectifs).**

| **TET** **(mg/kg/j)** | **Etifoxine** **(mg/kg/j)** | **J-3** **(moyenne ± esm)** | **J4** **(moyenne ± esm)** |
|---|---|---|---|
| **ACTIVITE AMBULATOIRE TOTALE** | | | |
| 0 | 0 | 2968 ± 353 | 2922 ± 276 |
| 3 | 0 | 2856 ± 320 | 1166 ± 114 * |
| 0 | 2 x 100 | 2950 ± 367 | 2345 ± 367 |
| 3 | 2 x 100 | 2659 ± 195 | 2174 ± 361 |

| **ACTIVITE AMBULATOIRE HORIZONTALE** | | | |
|---|---|---|---|
| 0 | 0 | 2310 ± 292 | 2269 ± 261 |
| 3 | 0 | 1839 ± 272 | 388 ± 71 * |
| 0 | 2 x 100 | 2195 ± 356 | 1714 ± 300 |
| 3 | 2 x 100 | 1998 ± 172 | 1650 ± 287 |

| **ACTIVITE AMBULATOIRE VERTICALE** | | | |
|---|---|---|---|
| 0 | 0 | 279 ± 48 | 331 ± 58 |
| 3 | 0 | 246 ± 48 | 2 ± 1 * |
| 0 | 2 x 100 | 284 ± 46 | 225 ± 39 * |
| 3 | 2 x 100 | 219 ± 20 | 162 ± 36 * |

**TABLEAU 6 : Effet de la déséthyl-étifoxine sur l'oedème cérébral induit par le triéthylétain (TET) en mesurant le pourcentage d'eau du cerveau, chez le rat (n = 10). Le rat perçoit, par voie orale pendant 5 jours, de la déséthyl-étifoxine 2 fois par jour et du TET à 3 mg/kg/j. Le 5^{ème} jour , le cerveau est prélevé puis desséché pendant 72 heures à 90°C, pour déterminer le pourcentage d'eau dans le cerveau.**

| **TET** **(mg/kg/j)** | **Déséthyl-étifoxine** **(mg/kg/j)** | **Pourcentage d'eau** **(moyenne ± esm)** | **Variation en pourcentage** | **Test statistique ANOVA** |
|---|---|---|---|---|
| 0 | 0 | 79,85 ± 0,03 | | |
| 3 | 0 | 81,29 ± 0,07 | + 1,44 | p < 0,05 |
| 0 | 2 x 50 | 79,76 ± 0,16 | - 0,09 | ns |
| 3 | 2 x 50 | 80,24 ± 0,12 | + 0,39 | ns |

**TABLEAU 7 : Effet de la déséthyl-étifoxine sur l'évolution du poids corporel perturbée par le triéthylétain (TET) chez le rat (n = 10). A partir de J0, le rat reçoit, par voie orale pendant 5 jours, de la déséthyl-étifoxine 2 fois par jour et du TET à 3 mg/kg/j. (*:p : < 0,05 par un test statistique ANOVA pour comparer les groupes traités au groupe sans traitement).**

| **TET** **(mg/kg/j)** | **Déséthyl-étifoxine** **(mg/kg/j)** | **Poids corporel en grammes (moyenne ± esm)** | | | | | |
|---|---|---|---|---|---|---|---|
| | | **J-3** | **J0** | **J1** | **J2** | **J3** | **J4** |
| 0 | 0 | 191±2 | 217±2 | 222±3 | 228±3 | 235±3 | 242±3 |
| 3 | 0 | 192±3 | 216±3 | 219±3 | 223±3 | 225±4 | 210±3 * |
| 0 | 2 x 50 | 193±3 | 219±3 | 224±3 | 232±3 | 240±3 | 246±3 |
| 3 | 2 x 50 | 194±3 | 222±3 | 226±4 | 230±4 | 238±4 | 241±3 |

**TABLEAU 8 : Effet de la déséthyl-étifoxine sur l'index neurologique perturbé par le triéthyétain (TET) en fonction du temps, chez le rat (n = 10). A partir de J0, le rat reçoit, par voie orale pendant 5 jours, de la déséthyl-étifoxine 2 fois par jour et du TET à 3 mg/kg/j. (*: p<0,05, test statistique ANOVA par rapport aux groupes témoins respectifs).**

| **TET** **(mg/kg/j)** | **Déséthyl-étifoxine** **(mg/kg/j)** | **Index neurologique (moyennes ± esm)** | | | | | |
|---|---|---|---|---|---|---|---|
| | | **J-3** | **J0** | **J1** | **J2** | **J3** | **J4** |
| 0 | 0 | 0 | 0 | 0 | 0 | 0 | 0 |
| 3 | 0 | 0 | 0 | 0 | 0 | 1,30±0,13 * | 2,35±0,13* |
| 0 | 2 x 50 | 0 | 0 | 0 | 0 | 0 | 0 |
| 3 | 2 x 50 | 0 | 0 | 0 | 0 | 0,20±0,13* | 0,15±0,08 |

| | | | | | | | |
|---|---|---|---|---|---|---|---|
| Index neurologique : 0 → pas d'anomalie apparente 1 → perte de l'activité spontanée : le rat ne sort pas d'une surface limitée dans un délai de 60 s, mais il s'en échappe normalement s'il est stimulé (bruit, pincement) ; il a perdu son activité exploratoire mais conserve ses capacités motrices 2 → perte du réflexe d'agrippement lorsque le rat est repoussé sur la surface 3 → perte du réflexe de retrait, coma suivi dans la plupart des cas de la mort | | | | | | | |

**TABLEAU 9 : Effet de la déséthyl-étifoxine sur l'activité ambulatoire (unité arbitraire) perturbée par le triéthylétain (TET) chez le rat (n = 10). Le rat perçoit, par voie orale pendant 5 jours, de la déséthyl-étifoxine 2 fois par jour et du TET à 3 mg/kg/j. Les mesures sont effectuées à J-3, avant tout traitement, et à J4, à la fin de l'étude. ( * : p<0,05, test statistique ANOVA par rapport aux groupes témoins respectifs).**

| **TET** **(mg/kg/j)** | **Déséthyl-étifoxine** **(mg/kg/j)** | J-3 **(moyenne ± esm)** | J4 **(moyenne ± esm)** |
|---|---|---|---|
| **ACTIVITE AMBULATOIRE TOTALE** | | | |
| 0 | 0 | 2558 ± 221 | 2606 ± 287 |
| 3 | 0 | 2782 ± 213 | 1445 ± 155 * |
| 0 | 2 x 50 | 2725 ± 364 | 2887 ± 423 |
| 3 | 2 x 50 | 2923 ± 210 | 2053 ± 256 |

| **ACTIVITE AMBULATOIRE HORIZONTALE** | | | |
|---|---|---|---|
| 0 | 0 | 1919 ± 171 | 1914 ± 224 |
| 3 | 0 | 2110 ± 181 | 601 ± 120 * |
| 0 | 2 x 50 | 2065 ± 298 | 2195 ± 355 |
| 3 | 2 x 50 | 2156 ± 182 | 1382 ± 222 |

| **ACTIVITE AMBULATOIRE VERTICALE** | | | |
|---|---|---|---|
| 0 | 0 | 259 ± 29 | 343 ± 43 |
| 3 | 0 | 320 ± 34 | 16 ± 7* |
| 0 | 2 x 50 | 327 ± 57 | 475 ± 96 |
| 3 | 2 x 50 | 328 ± 47 | 295 ± 64 |

## Revendications

1. Utilisation d'au moins un composé de formule (I) suivante : 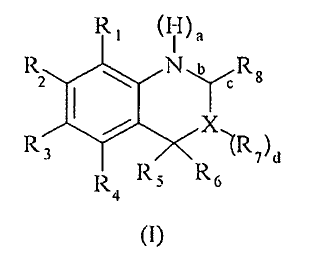 dans laquelle :
- a représente 0 ou 1 ;
- b représente une simple liaison ou une double liaison ;
- c représente une simple liaison ou une double liaison ;
- d représente 0 ou 1 ;
- X représente un atome d'oxygène ou d'azote, sous réserve que lorsque X représente un atome d'oxygène alors d vaut 0 et que lorsque X représente un atome d'azote alors d vaut 1 ;
- R₁, R₂, R₃, et R₄, identiques ou différents, représentent un atome d'hydrogène, un atome d'halogène, notamment choisi parmi F, Cl, Br, ou I, un groupe hydroxyle, ou un groupe alkoxyle de 1 ou 2 atomes de carbone ;
- R₅ et R₆, identiques ou différents, représentent un atome d'hydrogène, un groupe alkyle ou cycloalkyle de 1 à 6 atomes de carbone, ou un groupe aryle de 6 atomes de carbone dont le noyau aromatique est éventuellement substitué par un ou plusieurs atomes d'halogène ou un ou plusieurs groupes hydroxyle, alkoxyle de 1 ou 2 atomes de carbone, trifluorométhyle ou nitro ;
- R₇ représente un atome d'hydrogène, un groupe hydroxyle, ou un groupe alkyle ou hydroxyalkyle de 1 à 3 atomes de carbone ;
- R₈ représente un atome d'oxygène ou un groupe -NR₉R₁₀, R₉ et R₁₀, identiques ou différents, représentant un atome d'hydrogène, un groupe hydroxyle, ou un groupe alkyle ou hydroxyalkyle de 1 à 3 atomes de carbone, sous réserve que lorsque R₈ représente un atome d'oxygène alors a vaut 1, b représente une simple liaison et c représente un double liaison et que lorsque R₈ représente un groupe -NR₉R₁₀ alors a vaut 0, b représente une double liaison et c représente une simple liaison ;
ou de ses sels pharmaceutiquement acceptables,
pour la préparation d'un médicament destiné à la prévention ou au traitement des oedèmes cérébraux.

2. Utilisation selon la revendication 1, d'un composé de formule (II) suivante : 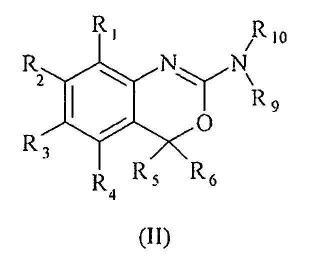 dans laquelle R₁, R₂, R₃, R₄, R₅, R₆, R₉ et R₁₀ sont tels que définis ci-dessus.

3. Utilisation selon la revendication 1 ou 2, des composés de formules (III) et (IV) suivantes :

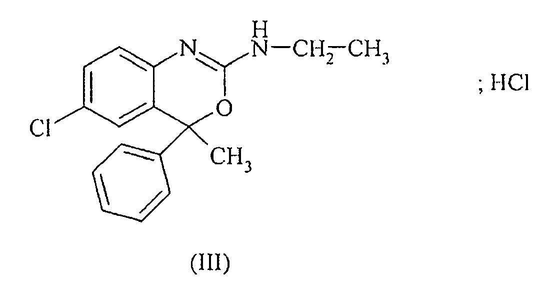

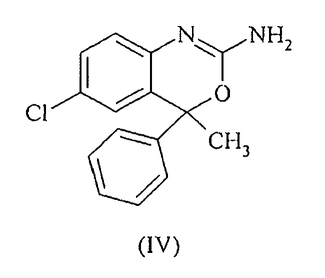

4. Utilisation selon la revendication 1, d'un composé de formule (V) suivante : 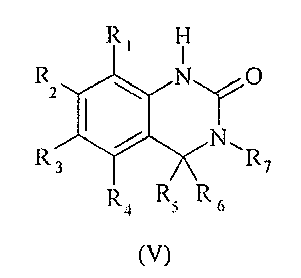 dans laquelle R₁, R₂, R₃, R₅, R₆, et R₇ sont tels que définis dans la revendication 1.

5. Utilisation selon la revendication 1 ou 4, de composés de formules (VI) et (VII) suivantes :

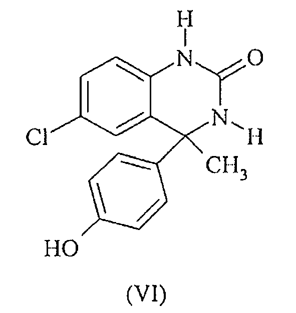

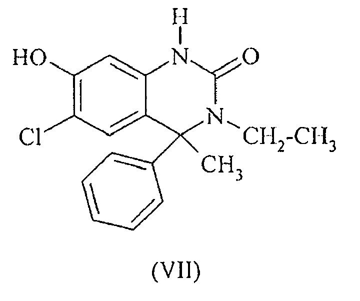

6. Utilisation selon l'une des revendications 1 à 5, dans laquelle l'oedème cérébral est consécutif à un accident vasculaire cérébral, un traumatisme cérébral, une tumeur cérébrale, des métastases cérébrales d'un cancer, un abcès cérébral, une poussée hypertensive, une acidocétose diabétique ou un neuropaludisme.

7. Utilisation selon l'une des revendications 1 à 6, dans laquelle le médicament convient pour l'administration à un individu en ayant besoin d'une dose unitaire d'environ 50 mg à environ 1500 mg, notamment d'environ 150 à 200 mg du composé tel que défini dans l'une des revendications 1 à 5.

8. Utilisation selon l'une des revendications 1 à 7, dans laquelle le médicament convient pour l'administration à un individu en ayant besoin d'une dose d'environ 50 mg/j à environ 1500 mg/j, notamment d'environ 150 mg/j à environ 200 mg/j, du compose tel que défini dans l'une des revendications 1 à 5.

9. Utilisation selon l'une des revendications 1 à 8, dans laquelle le médicament convient pour une administration par voie orale.

10. Utilisation selon l'une des revendications 1 à 9, dans laquelle le médicament se présente sous la forme d'une poudre, de cachets, de gélules ou de sachets.

11. Utilisation selon l'une des revendication 1 à 10, dans laquelle le composé tel que défini dans l'une des revendications 1 à 5 est associé à au moins un composé additionnel destiné à la prévention ou au traitement de l'oedème cérébral, tel qu'un composé choisi parmi la liste comprenant un corticoïde, notamment un glucocorticoïde, du glycérol, du mannitol, un diurétique, notamment le furosémide, un barbiturique, le tétracosactide, un antibiotique, la CDP-choline, la vinpocétine, un inhibiteur calcique, et un antagoniste NMDA.

12. Composition pharmaceutique, comprenant à titre de substance active, au moins un composé de formule (I) tel que défini dans l'une des revendications 1 à 5, ou un sel pharmaceutiquement acceptable de celui-ci, et au moins un composé additionnel destiné à la prévention ou au traitement de l'oedème cérébral, tel qu'un composé choisi parmi la liste comprenant un corticoïde, notamment un glucocorticoïde, du glycérol, du mannitol, un diurétique, notamment le furosémide, un barbiturique, le tétracosactide, un antibiotique, la CDP-choline, la vinpocétine, un inhibiteur calcique, un antagoniste NMDA, en association avec un véhicule pharmaceutiquement acceptable.

13. Produits contenant :
- au moins un composé de formule (I) tel que défini dans l'une des revendications 1 à 5, ou un sel pharmaceutiquement acceptable de celui-ci, et
- au moins un composé additionnel destiné à la prévention ou au traitement de l'oedème cérébral, tel qu'un composé choisi parmi la liste comprenant un corticoïde, notamment un glucocorticoïde, du glycérol, du mannitol, un diurétique, notamment le furosémide, un barbiturique, le tétracosactide, un antibiotique, la CDP-choline, la vinpocétine, un inhibiteur calcique, et un antagoniste NMDA,
comme produit de combinaison pour une utilisation simultanée, séparée ou étalée dans le temps pour la prévention ou le traitement de l'oedème cérébral.

14. Composé de formule (I), (II), (III), (IV), (V), (VI), ou (VII), tel que défini dans l'une des revendications 1 à 5, pour son utilisation dans la prévention ou le traitement de l'oedème cérébral.

15. Composé de formule (1), (II), (III), (IV), (V), (VI), ou (VII), tel que défini dans l'une des revendications 1 à 5, pour son utilisation selon la revendication 14, dans laquelle l'oedème cérébral est consécutif à un accident vasculaire cérébral, un traumatisme cérébral, une tumeur cérébrale, des métastases cérébrales d'un cancer, un abcès cérébral, une poussée hypertensive, une acidocétose diabétique ou un neuropaludisme.

16. Composé de formule (I), (II), (III), (IV), (V), (VI), ou (VII), tel que défini dans l'une des revendications 1 à 5, pour son utilisation selon la revendication 14 ou 15, dans laquelle le composé tel que défini dans l'une des revendications 1 à 5 est associé à au moins un composé additionnel destiné à la prévention ou au traitement de l'oedème cérébral, tel qu'un composé choisi parmi la liste comprenant un corticoïde, notamment un glucocorticoïde, du glycérol, du mannitol, un diurétique, notamment le furosémide, un barbiturique, le tétracosactide, un antibiotique, la CDP-choline, la vinpocétine, un inhibiteur calcique, et un antagoniste NMDA.

## Claims

1. The use of at least one compound of formula (I) below: 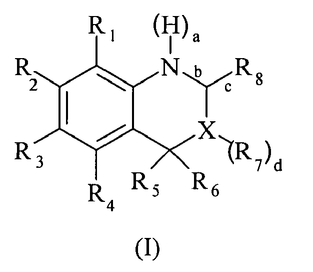 in which:
- a represents 0 or 1;
- b represents a single bond or a double bond;
- c represents a single bond or a double bond;
- d represents 0 or 1;
- X represents an oxygen atom or a nitrogen atom, with the proviso that when X represents an oxygen atom, d is 0 and when X represents a nitrogen atom, d is 1;
- R₁, R₂, R₃ and R₄, which may be identical or different, represent a hydrogen atom, a halogen atom, especially selected from F, Cl, Br and I, a hydroxyl group, or an alkoxy group having 1 or 2 carbon atoms;
- R₅ and R₆, which may be identical or different, represent a hydrogen atom, an alkyl or cycloalkyl group having from 1 to 6 carbon atoms, or an aryl group having 6 carbon atoms, the aromatic ring of which is optionally substituted by one or more halogen atoms or by one or more hydroxyl, alkoxy having 1 or 2 carbon atoms, trifluoromethyl or nitro groups;
- R₇ represents a hydrogen atom, a hydroxyl group, or an alkyl or hydroxyalkyl group having from 1 to 3 carbon atoms;
- R₈ represents an oxygen atom or a -NR₉R₁₀ group, R₉ and R₁₀, which may be identical or different, representing a hydrogen atom, a hydroxyl group, or an alkyl or hydroxyalkyl group having from 1 to 3 carbon atoms, with the proviso that when R₈ represents an oxygen atom, a is 1, b represents a single bond and c represents a double bond, and when R₈ represents a group - NR₉R₁₀, a is 0, b represents a double bond and c represents a single bond;
or of the pharmaceutically acceptable salts thereof,
in the preparation of a medicament for the prevention or treatment of cerebral oedemas.

2. The use according to claim 1 of a compound of formula (II) below: 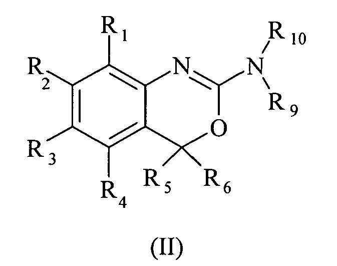 in which R₁, R₂, R₃, R₄, R₅, R₆, R₉ and R₁₀ are as defined hereinbefore.

3. The use according to claim 1or 2 of compounds of formulae (III) and (IV) below:

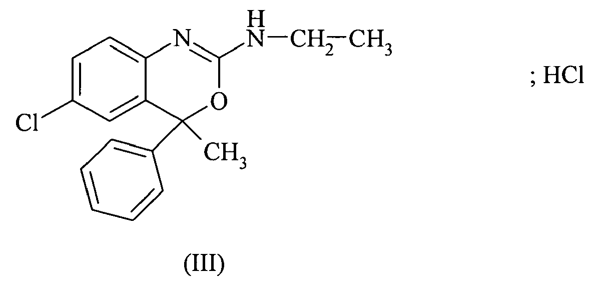

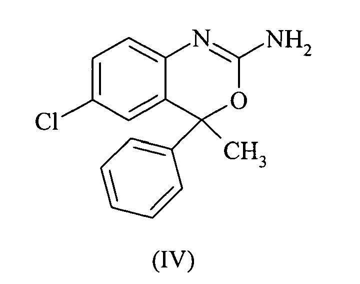

4. The use according to claim 1 of a compound of formula (V) below: 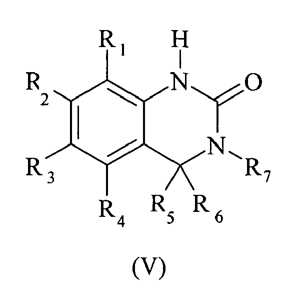 in which R₁, R₂, R₃, R₅, R₆ and R₇ are as defined in claim 1.

5. The use according to claim 1 or 4 of compounds of formulae (VI) and (VII) below:

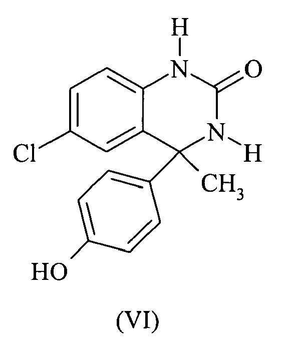

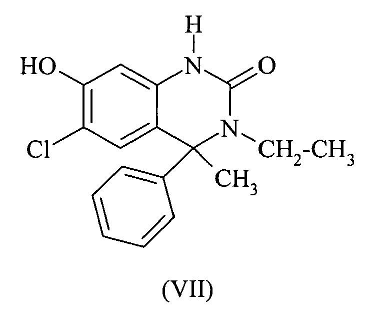

6. The use according to any of claims 1 to 5, in which the cerebral oedema follows a cerebral vascular accident, a cerebral trauma, a cerebral tumour, cerebral metastases of a cancer, a cerebral abscess, a hypertensive attack, a diabetic ketoacidosis or a neuropaludism.

7. The use according to any of claims 1 to 6, in which the medicament is suitable for administration to an individual requiring a unit dose of from approximately 50 mg to approximately 1500 mg, especially of approximately from 150 to 200 mg, of the compound as defined in any of claims 1 to 5.

8. The use according to claims 1 to 7, in which the medicament is suitable for administration to an individual in need thereof of a dose of from approximately 50 mg/day to approximately 1500 mg/day, especially from approximately 150 mg/day to approximately 200 mg/day, of the compound as defined in any of claims 1 to 5.

9. The use according to any of claims 1 to 8, in which the medicament is suitable for oral administration.

10. The use according to any of claims 1 to 9, in which the medicament is in the form of a powder, tablets, gelatine capsules or sachets.

11. The use according to any of claims 1 to 10, in which the compound as defined in any of claims 1 to 5 is associated with at least one additional compound for the prevention or treatment of cerebral oedema, such as a compound selected from the list comprising a corticoid, especially a glucocorticoid, glycerol, mannitol, a diuretic, especially furosemide, a barbiturate, tetracosactide, an antibiotic, CDP-choline, vinpocetine, a calcium inhibitor and a NMDA antagonist.

12. A pharmaceutical composition comprising as active ingredient at least one compound of formula (I) as defined in any of claims 1 to 5, or a pharmaceutically acceptable salt thereof, and at least one additional compound for the prevention or treatment of cerebral oedema, such as a compound selected from the list comprising a corticoid, especially a glucocorticoid, glycerol, mannitol, a diuretic, especially furosemide, a barbiturate, tetracosactide, an antibiotic, CDP-choline, vinpocetine, a calcium inhibitor and a NMDA antagonist, in association with a pharmaceutically acceptable carrier.

13. Products comprising:
- at least one compound of formula (I) as defined in claim 1, or a pharmaceutically acceptable salt thereof, and
- at least one additional compound for the prevention or treatment of cerebral oedema, such as a compound selected from the list comprising a corticoid, especially a glucocorticoid, glycerol, mannitol, a diuretic, especially furosemide, a barbiturate, tetracosactide, an antibiotic, CDP-choline, vinpocetine, a calcium inhibitor and a NMDA antagonist,
as a combination product for simultaneous, separate or sequential use in the prevention or treatment of cerebral oedema.

14. A compound of formula (I), (II), (III), (IV), (V), (VI), or (VII), as defined in any of claims 1 to 5, for use in the prevention or treatment of cerebral oedema.

15. The compound of formula (I), (II), (III), (IV), (V), (VI), or (VII), as defined in any of claims 1 to 5, for use according to claim 14, wherein the cerebral oedema follows a cerebral vascular accident, a cerebral trauma, a cerebral tumour, cerebral metastases of a cancer, a cerebral abscess, a hypertensive attack, a diabetic ketoacidosis or a neuropaludism.

16. The compound of formula (I), (II), (III), (IV), (V), (VI), or (VII), as defined in any of claims 1 to 5, for use according to claim 14 or 15, wherein the compound as defined in any of claims 1 to 5 is associated with at least one additional compound for the prevention or treatment of cerebral oedema, such as a compound selected from the list comprising a corticoid, especially a glucocorticoid, glycerol, mannitol, a diuretic, especially furosemide, a barbiturate, tetracosactide, an antibiotic, CDP-choline, vinpocetine, a calcium inhibitor and a NMDA antagonist.

## Patentansprüche

1. Verwendung wenigstens einer Verbindung der folgenden Formel (I): 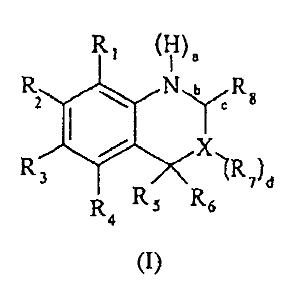 in der:
- a 0 oder 1 darstellt;
- b eine Einfachbindung oder eine Doppelbindung darstellt;
- c eine Einfachbindung oder eine Doppelbindung darstellt;
- d 0 oder 1 darstellt;
- X ein Sauerstoff- oder Stickstoffatom darstellt, mit der Maßgabe, dass, wenn X ein Sauerstoffatom darstellt, d dann 0 ist und, wenn X ein Stickstoffatom darstellt, d dann 1 ist;
- R₁, R₂, R₃ und R₄, die gleich oder unterschiedlich sind, ein Wasserstoffatom, ein Halogenatom, insbesondere ausgewählt aus F, Cl, Br und I, oder eine Hydroxylgruppe oder eine Alkoxylgruppe mit 1 oder 2 Kohlenstoffatomen darstellen;
- R₅ und R₆, die gleich oder verschieden sind, ein Wasserstoffatom, eine Alkyl- oder Cycloalkylgruppe mit 1 bis 6 Kohlenstoffatomen oder eine Arylgruppe mit 6 Kohlenstoffatomen, deren aromatischer Ring gegebenenfalls mit einem oder mehreren Halogenatomen oder einer oder mehrere Hydroxylgruppe(n), Alkoxylgruppe(n) mit 1 oder 2 Kohlenstoffatomen, Trifluormethyl oder Nitro substituiert ist, darstellen;
- R₇ ein Wasserstoffatom, eine Hydroxylgruppe oder eine Alkyl- oder Hydroxyalkylgruppe mit 1 bis 3 Kohlenstoffatomen darstellt;
- R₈ ein Sauerstoffatom oder eine Gruppe -NR₉R₁₀ darstellt, wobei R₉ und R₁₀, die gleich oder unterschiedlich sind, ein Wasserstoffatom, eine Hydroxylgruppe oder eine Alkyl- oder Hydroxyalkylgruppe mit 1 bis 3 Kohlenstoffatomen darstellen, mit der Maßgabe, dass, wenn R₈ ein Sauerstoffatom darstellt, dann a 1 ist, b eine Einfachbindung darstellt und c eine Doppelbindung darstellt, und dass, wenn R₈ eine Gruppe -NR₉R₁₀ darstellt, dann a 0 ist, b eine Doppelbindung darstellt und c eine Einfachbindung darstellt;
und ihrer pharmazeutisch verträglichen Salze für die Herstellung eines Medikaments, das zur Prävention oder Behandlung von Gehirnödemen bestimmt ist.

2. Verwendung nach Anspruch 1 einer Verbindung der folgenden Formel (II): 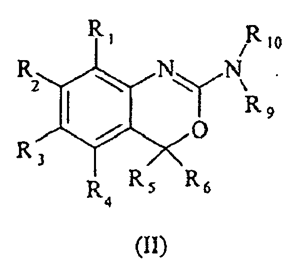 in der R₁, R₂, R₃, R₄, R₅, R₆, R₉ und R₁₀ wie oben definiert sind.

3. Verwendung nach Anspruch 1 oder 2 der Verbindungen der folgenden Formeln (III) und (IV):

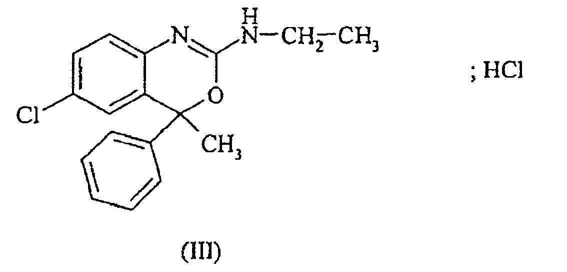

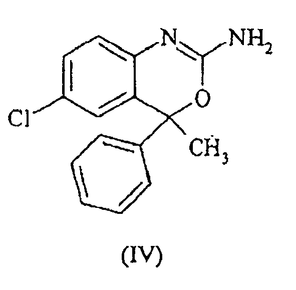

4. Verwendung nach Anspruch 1 einer Verbindung der folgenden Formel (V): 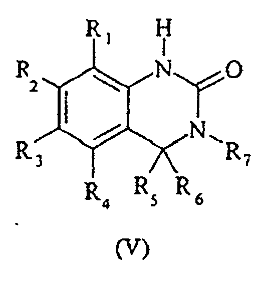 in der R₁, R_{2,} R₃, R₅, R₆ und R₇ wie in Anspruch 1 definiert sind.

5. Verwendung nach Anspruch 1 oder 4 der Verbindungen der folgenden Formeln (VI) und (VII):

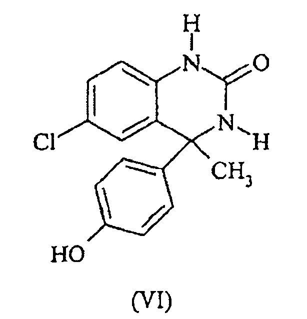

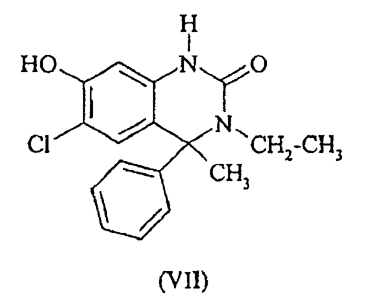

6. Verwendung nach einem der Ansprüche 1 bis 5, wobei das Gehirnödem Folge eines cerebrovaskulären Insults, eines Gehirntraumas, eines Gehirntumors, von Gehirnmetastasen, von Krebs, eines Gehirnabszesses, von Bluthochdruck, diabetischer Ketoazidose oder Neuromalaria ist.

7. Verwendung nach einem der Ansprüche 1 bis 6, bei der das Medikament für die Verabreichung an ein Individuum, das einer solchen bedarf, mit einer Einheitsdosis von etwa 50 mg bis etwa 1.500 mg, insbesondere etwa 150 bis 200 mg der Verbindung, wie sie in einem der Ansprüche 1 bis 5 definiert ist, geeignet ist.

8. Verwendung nach einem der Ansprüche 1 bis 7, wobei das Medikament für die Verabreichung an ein Individuum, das einer solchen bedarf, einer Dosis von etwa 50 mg/Tag bis etwa 1500 mg/Tag, insbesondere etwa 150 mg/Tag bis etwa 200 mg/Tag der Verbindung, wie sie in einem der Ansprüche 1 bis 5 definiert ist, geeignet ist.

9. Verwendung nach einem der Ansprüche 1 bis 8, wobei das Medikament für eine Verabreichung auf oralem Weg geeignet ist.

10. Verwendung gemäß einem der Ansprüche 1 bis 9, wobei das Medikament in Form eines Pulvers, von Cachets, Kapseln oder Briefchen vorliegt.

11. Verwendung gemäß einem der Ansprüche 1 bis 10, wobei die Verbindung, wie sie in einem der Ansprüche 1 bis 5 definiert ist, mit wenigstens einer zusätzlichen Verbindung kombiniert ist, die zur Prävention oder Behandlung des Gehirnödems bestimmt ist, zum Beispiel eine Verbindung, ausgewählt aus der Liste, umfassend ein Corticoid, insbesondere ein Glucocorticoid, Glycerin, Mannit, ein Diuretikum, insbesondere Furosemid, ein Barbiturat, Tetracosactid, ein Antibiotikum, CDP-Cholin, Vinpocetin, einen Calciumkanalblocker und einen NMDA-Antagonist.

12. Pharmazeutische Zusammensetzung, umfassend als aktive Substanz wenigstens eine Verbindung der Formel (I), wie sie in einem der Ansprüche 1 bis 5 definiert ist, oder ein pharmazeutisch verträgliches Salz davon und wenigstens eine zusätzliche Verbindung, bestimmt zur Prävention oder Behandlung von Gehirnödem, wie eine Verbindung, ausgewählt aus der Liste, umfassend ein Corticoid, insbesondere ein Glucocorticoid, insbesondere ein Glucocorticoid, Glycerin, Mannit, ein Diuretikum, insbesondere Furosemid, ein Barbiturat, Tetracosactid, ein Antibiotikum, CDP-Cholin, Vinpocetin, einen Calciumkanalblocker, einen NMDA-Antagonist, in Verbindung mit einem pharmazeutisch verträglichen Vehikel.

13. Produkte, enthaltend:
- wenigstens eine Verbindung der Formel (I), wie sie in einem der Ansprüche 1 bis 5 definiert ist, oder ein pharmazeutisch verträgliches Salz davon und
- wenigstens eine zusätzliche Verbindung, bestimmt zur Prävention oder Behandlung von Gehirnödem, wie zum Beispiel eine Verbindung, ausgewählt aus der Liste, umfassend ein Corticoid, insbesondere ein Glucocorticoid, insbesondere ein Glucocorticoid, Glycerin, Mannit, ein Diuretikum, insbesondere Furosemid, ein Barbiturat, Tetracosactid, ein Antibiotikum, CDP-Cholin, Vinpocetin, einen Calciumkanalblocker, einen NMDA-Antagonist,
als Kombinationsprodukt für eine gleichzeitige, getrennte oder über die Zeit verteilte Verwendung zur Prävention oder Behandlung von Gehirnödem.

14. Verbindung der Formel (I), (II), (III), (IV), (V), (VI) oder (VII), wie in einem der Ansprüche 1 bis 5 definiert, zur Verwendung zur Prävention oder zur Behandlung des Gehirnödems.

15. Verbindung der Formel (I), (II), (III), (IV), (V), (VI) oder (VII), wie in einem der Ansprüche 1 bis 5 definiert, zur Verwendung nach Anspruch 14, wobei das Gehirnödem die Folge eines cerebrovaskulären Insults, eines Gehirntraumas, eines Gehirntumors, von Gehirnmetastasen von Krebs, eines Gehirnabszesses, von Bluthochdruck, diabetischer Ketoazidose oder Neuromalaria ist.

16. Verbindung der Formel (I), (II), (III), (IV), (V), (VI) oder (VII), wie in einem der Ansprüche 1 bis 5 definiert, zur Verwendung nach Anspruch 14 oder 15, wobei die Verbindung, wie sie in einem der Ansprüche 1 bis 5 definiert ist, mit wenigstens einer zusätzlichen Verbindung kombiniert ist, die zur Prävention oder Behandlung des Gehirnödems bestimmt ist, zum Beispiel eine Verbindung, ausgewählt aus der Liste, umfassend ein Corticoid, insbesondere ein Glucocorticoid, Glycerin, Mannit, ein Diuretikum, insbesondere Furosemid, ein Barbiturat, Tetracosactid, ein Antibiotikum, CDP-Cholin, Vinpocetin, ein Calciumkanalblocker und ein NMDA-Antagonist.
